# EUROPÄISCHE PATENTSCHRIFT

(11) **EP 0 566 056 B1**
(45) Veröffentlichungstag und Bekanntmachung des Hinweises auf die Patenterteilung: **17.01.1996**
(21) Anmeldenummer: 93105946.3
(22) Anmeldetag: 13.04.1993
(51) Int. Cl.: C02F 3/28

(54) **Verfahren zur biologischen Aufbereitung organischer Substanzen, insbesondere zur anaeroben biologischen Hydrolyse zur anschliessenden Biomethanisierung, und Vorrichtungen zur Durchführung des Verfahrens**
Processes and apparatusses for biological treatment of organic substances, especially for anaerobic biological hydrolysis for the following biomethanation
Procédés et dispositifs pour le traitement biologique de substances organiques, en particulier pour l'hydrolyse biologique anaerobic à la biométhanisation suivante

(30) Priorität: 16.04.1992 DE 4212869; 06.08.1992 DE 4226087
(43) Veröffentlichungstag der Anmeldung: 20.10.1993
(73) Patentinhaber: REA GESELLSCHAFT FÜR RECYCLING VON ENERGIE UND ABFALL MBH, D-80333 München (DE)
(72) Erfinder: Kübler, Hans, W-8000 München 2 (DE)
(74) Vertreter: Reinhard - Skuhra - Weise & Partner

(56) Entgegenhaltungen:
- EP-A- 0 048 675
- EP-A- 0 351 394
- WO-A-92/13084
- US-A- 4 318 993
- PATENT ABSTRACTS OF JAPAN vol. 6, no. 122 (C-112)(1000) 7. Juli 1982

## Beschreibung

Die Erfindung betrifft Verfahren zur biologischen Aufbereitung organischer Substanzen, insbesondere zur anaeroben biologischen Hydrolyse zur anschließenden Biomethanisierung, sowie Vorrichtungen zur Durchführung der Verfahren.

Die Biomethanisierung von komplexen organischen Materialien erfolgt durch das Zusammenspiel von hauptsächlich drei Mikroorganismengruppen:
1. Hydrolytische fermentative Bakterien
2. Wasserstoffproduzierende acetogene Bakterien
3. Wasserstoff und Acetat verbrauchende methanogene Bakterien.

Hierbei werden die Produkte der ersten Gruppe von der zweiten und die Produkte der zweiten von der dritten Mikroorganismengruppe verwertet. Dadurch entstehen aus komplexen Biopolymeren die Hauptprodukte Methan und Kohlendioxid.

Fermentative Mikroorganismen verwerten zur Energiegewinnung bevorzugt einfache organische Substanzen wie z.B. Glucose oder Cellubiose, denn diese sind gelöst und können somit direkt resorbiert werden. Unter anaeroben Bedingungen sind dabei die Fermentationsprodukte hauptsächlich organische Säuren oder Alkohole.

Dagegen können komplexe organische Substanzen wie z.B. Cellulose nicht direkt resorbiert werden. Sie müssen zuerst in resorbierbare Bruchstücke hydrolysiert werden. Dazu scheiden hydrolytisch aktive Mikroorganismen spezifische Exoenzyme aus, die die Biopolymere zerlegen. Die Spaltprodukte werden im anaeroben Milieu daraufhin zu organischen Säuren oder Alkoholen fermentiert.

Da die Fermentation gelöster Substanzen für die Mikroorganismen energetisch erheblich günstiger ist, bilden sie hydrolytische Exoenzyme erst bei Abwesenheit von gelösten fermentierbaren Stoffen (Buchholz, K. und H.-J. Arntz (1988): Gewinnung von Enzymen durch anaerobe Fermentation von Rübenpreßschnitzeln, Zuckerindustrie 113 (1988), S. 204-208). Das bedeutet, daß bei einem Substratgemisch aus gelösten und nichtgelösten Substanzen zuerst die gelösten Stoffe weitgehend fermentiert werden, bevor die Biopolymere hydrolysiert und fermentiert werden.

Bei einer Anreicherung der Produkte im Medium kommt es zu einer Hemmung des Fermentationsprozesses. Verstärkt wird diese Produkthemmung durch den pH-Wertabfall, der durch die gebildeten Säuren verursacht wird. Besonders bei pH-Werten unter 6 ist wegen der geringen Dissoziation der gebildeten organischen Säuren sehr rasch ein hemmendes Konzentrationsniveau erreicht.

Die Aktivität der hydrolytischen Enzyme ist ebenfalls stark vom pH-Wert im Medium abhängig. Die meisten hydrolytischen Enzyme anaerober Mikroorganismen haben ihre größte Aktivität im pH-Bereich von 6 bis leicht über 7 (Rodriguez, H., O. Volfova und A. Klyosov: Characterization of the cellulose complex from Cellulomonas grown on bagasse pitch, App. Microbiol. Biotechnolo. 28 (1988), S. 394-397).

Um einer Anreicherung der gebildeten Säuren und einem pH-Wertabfall entgegenzuwirken, müssen die Säuren eliminiert werden. Im Gegensatz zu einer einstufigen Verfahrensführung, bei der sie im selben Reaktor sofort methanisiert werden, müssen bei mehrstufigen Verfahren die in der Hydrolyse- und Versäuerungsstufe gebildeten Säuren gezielt abgeführt und der Methanisierungsstufe zugeführt werden. Diesem Nachteil der mehrstufigen Verfahrensführung steht jedoch deren höhere Leistungsfähigkeit entgegen (Bailey, J.E. und D.F. Ollis; Biochemical Engineering Fundamentals (McGraw-Hill, New York 1977)).

Nach Noike (Noike, T., u.a.: Characteristics of carbohydrate degradation and the ratelimiting step in anaerobic digestion, Biotechnology and Bioengineering 27 (1985), S. 1482-1489) ist bei der Biomethanisierung von organischen Feststoffen die anaerobe Hydrolyse der Feststoffe der geschwindigkeitsbestimmende Schritt. Durch Einstellen optimaler Milieubedingungen kann der Stoffumsatz der hydrolytischen Mikroorganismen gesteigert und somit der limitierende Abbauschritt beschleunigt werden. Für viele anaerobe hydrolytische Mikroorganismen liegt der optimale pH-Wert im sauren Bereich.

Für die Biomethanisierung der Hydrolyseprodukte ist jedoch ein neutraler pH-Wert günstiger. Im sauren Bereich nimmt der Stoffumsatz methanogener Populationen stärker ab. Dadurch wird bei pH-Werten, die für die Hydrolyse optimal sind, die Methanisierung der Hydrolyseprodukte zum geschwindigkeitsbestimmenden Schritt. Durch Trennung der beiden Abbauschritte durch eine zweistufige Prozessführung werden für beide Abbauschritte optimale Rahmenbedingungen geschaffen.

Bei kontinuierlichem Zufluß von komplexen Substraten, wie z.B. Abfallgemischen zur Versäuerungsstufe, ist wegen der ständigen Zufuhr an leicht vergärbaren Substanzen (meist gelöst) die Hydrolyse der Biopolymere gehemmt. Durch eine zweistufige Prozessführung mit Versäuerung und anschließender Hydrolyse wird bei kontinuierlicher Substratzufuhr diese Hemmung unterbunden. Die gelösten und leicht vergärbaren Substanzen werden in der ersten Stufe versäuert und der Hydrolysestufe werden nur noch Feststoffe zugeführt. Durch diesen Selektionsdruck bildet sich in der zweiten Stufe eine sehr aktive, hydrolytische Population aus, so daß der Feststoffabbau gesteigert ist.

Gonzales (Gonzales, G., G. Caminal, C. de Mas und J. Lopez-Santin: A kinetic model for pretreated wheat straw saccharification by cellulase, Journal Chem. Tech. Biotechnol. (1989), S. 275) zeigen, daß Enzymreaktionen zum Abbau von Cellulose durch Michaelis-Menten Reaktionen beschrieben werden können. Das bedeutet, daß hohe Substratkonzentrationen auf die Hydrolyse einen günstigen Einfluß haben. Jedoch verändert sich mit zunehmender Feststoffkonzentration die Rheologie im Reaktor und durch eine Transportlimitierung nehmen die Reaktionsgeschwindigkeiten ab. Somit existiert für die Feststoffhydrolyse in Abhängigkeit vom jeweiligen Substratgemisch eine optimale Feststoffkonzentration.

Bisher jedoch wurden keine derart optimierten Verfahren angegeben. Bei dem in der US-PS 4,781,836 beschriebenen Verfahren zur Biomethanisierung organischer Substanzen mit zwei Verarbeitungsstufen erfolgt die Versäuerung der gelösten Bestandteile und die Hydrolyse der nichtgelösten Substanzen in ein und demselben Reaktor in einem für die dort stattfindenden Prozesse nicht optimalen Milieu. Zudem sind die Wasserkreisläufe der beiden Reaktoren völlig voneinander getrennt. Diese Trennung erfolgt durch eine Kombination von Filter zur Fest-/Flüssig-Trennung und Ionenaustauscher zur Abtrennung und Übergabe der gelösten polaren Substanzen. Eine derartige Verfahrensführung ist für die Behandlung feststoffhaltiger Gemische aus komplexen Substanzen ungeeignet, da entsprechende Filter entweder nicht mit dem versäuerten Gemisch beschickt werden können oder das Filtrat für die Beschickung eines Ionenaustauschers zu feststoffhaltig ist. In der EP-A-0 351 394 ist ein Verfahren zur Erhöhung der Methanausbeute bei der Vergärung kommunaler organischer Abfälle mit zwei getrennten Stufen und zwei dabei involvierten gemischten Gärungsstufen in einem ersten und einem zweiten Reaktor beschrieben, bei welchem jeweils in den beiden Reaktoren eine Säuerung und Methanisierung der kommunalen organischen Abfälle vorgenommen wird. Auch bei diesen Verfahren erfolgt die Versäuerung der gelösten Bestandteile und die Hydrolyse der nichtgelösten Substanzen in ein und demselben Reaktor unter nicht optimalen Bedingungen.

Buchholz (1986), Gijzen und Zwart (Buchholz, K., H.-J. Arntz, A. Pellegrini und E. Stoppok (1986): Untersuchung zur Bildung von Biogas aus Rübenpreßschnitzeln, Zuckerindustrie 111 (1986), S. 837-844; Gijzen, H.J., u.a.: High-rate two-phase-process for the anaerobic degradation of cellulose, employing rumen mikroorganisms for an efficient acidogenesis, Biotechnology and Bioengineering 31 (1988), S. 418-425; Zwart, K.B., u.a.: Anaerobic digestion of a cellulosic fraction of domestic refuse by a two-phase rumen-derived process, Biotechnology and Bioengineering 32 (1988), S. 729-724) beschreiben in ihren Veröffentlichungen ein zweistufiges Verfahren zur anaeroben Vergärung von organischen Feststoffen. Auch in diesem Verfahren erfolgt die Versäuerung der gelösten Bestandteile und die Hydrolyse der nichtgelösten Substanzen in einem Reaktor.

Diese gemeinsame Versäuerung und Hydrolyse hat den Nachteil, daß die Bildung der hydrolytischen Exoenzyme solange unterdrückt wird, bis die gelösten und leicht vergärbaren Substanzen vollständig versäuert sind. Bei einer kontinuierlichen Zufuhr von Substrat stellt sich im Reaktor in Abhängigkeit der Umsatzraten eine Gleichgewichtskonzentration an unversäuerten und leicht vergärbaren Substanzen ein. Dies hat zur Folge, daß die Bildung der Exoenzyme und damit die Feststoffhydrolyse gehemmt ist.

Nur durch eine diskontinuierliche Beschickung des ersten Reaktors kann bei diesem zweistufigen Verfahren die Konzentration an leicht vergärbaren Substanzen zeitweise auf so geringe Werte gedrückt werden, daß eine gute Hydrolyse der schwer abbaubaren Feststoffe erfolgt.

In der Veröffentlichung von Hack, P.J.F.M. und Brinkmann, J. A: New Process for High Performance Digestion, International Symposium on Anearobic Digestion of Solid Waste, Venedig 14. - 17.4.92, wird eine dreistufige Verfahrensführung mit den Schritten Versäuerung - Hydrolyse - Methanisierung vorgeschlagen, wobei die einzelnen Verfahrensschritte in räumlich voneinander getrennten Reaktoren durchgeführt werden, und wobei die Rückführung des Ablaufs der Methanisierung zur Steuerung des pH-Werts und der Feststoffkonzentration eingesetzt wird. In diesem im folgenden kurz PAQUES genannten Verfahren werden nach einem mechanischen Vorbehandeln die Feststoffe hydrolysiert und in einem Reaktor 1 (Prethane-Reaktor) angesäuert, wobei die schnell abbaubaren Feststofffraktionen in Lösung gehen. Der resultierende Slurry wird getrennt, und die Hauptmenge der Feststofffraktion wieder in den Prethane-Reaktor zur weiteren Hydrolyse rückgeführt. Nur ein kleiner Teil der Feststoffe wird in den zweiten Reaktor (RUDAD-Reaktor) überführt.

In diesem zweiten Reaktor wird insbesondere durch Ciliaten und anaerobe Pilze die Feststofffraktion hydrolysiert und Cellulose und andere faserförmige Verbindungen werden angesäuert. Die Endprodukte dieser Hydrolyseverfahren sind insbesondere flüchtige Fettsäuren. Die nicht-abbaubaren Feststoffe werden aus dem RUDAD-Reaktor ausgeführt.

In einer dritten Stufe werden in einem dritten Reaktor der Flüssiganteil aus dem Reaktor 1 und die Hydrolyseendprodukte aus dem Reaktor 2 methanisiert.

Der anaerobe Ablauf aus dem dritten Reaktor wird im Prethane- und im RUDAD-Reaktor zur Verdünnung und zur pH-Wert-Kontrolle verwendet.

Dieses PAQUES-Verfahren hat den Nachteil, daß die drei insbesondere prozeßrelevanten Parameter, nämlich die pH-Wert-Steuerung, die Feststoffkonzentration im Reaktor 2 und die Feststoffverweilzeit im Reaktor 2 nur in Abhängigkeit voneinander einstellbar sind, so daß eine definierte Einstellung aller drei Parameter zur Steuerung der Geschwindigkeit der Feststoffhydrolyse wesentlich erschwert ist. Ein weiterer Nachteil des PAQUES-Verfahrens ist die Rückführung des größten Teils des Feststoffstroms nach der Trennung in den Reaktor 1 (Prethane-Reaktor). Diese Verfahrensführung erfordert unter anderem die Trennung, der Stoffströme in zwei Teilströme und damit einen höheren maschinentechnischen Aufwand. Weiterhin nachteilig ist der Feststoffabbau durch Ciliaten und anaerobe Pilze im Reaktor 2. Da diese Mikroorganismen meistens nicht in den so verarbeiteten Feststoffen vorhanden sind, ist das Einfahren des Reaktors 2 mit einem speziellen Impfschlamm erforderlich, wodurch das Verfahren verteuert wird.

Es ist eine Aufgabe der Erfindung, die aus dem Stand der Technik bekannten Verfahren einfacher und effizienter auszugestalten, insbesondere ein Steuerungskonzept bereitzustellen, durch welches der pH-Wert, des Reaktors 2 regulierbar ist.

Eine weitere Aufgabe der Erfindung ist es, eine zur Durchführung des erfindungsgemäßen Verfahrens geeignete Vorrichtung anzugeben. Diese Aufgaben werden erfindungsgemäß durch die in den Ansprüchen 1 und 29 aufgeführten Merkmale gelöst.

Zur besseren Separation der gelösten von den nichtgelösten organischen Substanzen werden in einem eigenen Verfahrensschritt die dem ersten Reaktor 1 direkt entnommenen gelösten und nichtgelösten organischen Substanzen einer Trennung in eine Feststofffraktion mit den nichtgelösten organischen Substanzen und eine Flüssigfraktion mit den gelösten organischen Substanzen unterzogen.

Der Vorteil einer getrennten Versäuerungs- und Hydrolysestufe besteht darin, daß verfahrensbedingt nur weitgehendst versäuertes Material (d.h. nur ein geringer Anteil an leicht fermentierbaren Substanzen) der Hydrolysestufe zugeführt wird, und somit auch bei kontinuierlicher Zufuhr der organischen Substanzen eine ungehemmte Feststoffhydrolyse gewährleistet wird.

Erfindungsgemäß wird der Hauptteil des Feststoffstroms aus dem Reaktor 1 in den Reaktor 2 überführt, wobei keine Rückführung der nichtgelösten Substanzen in den Reaktor 1 erfolgt. Im PAQUES-Verfahren wird jedoch im Gegensatz zum erfindungsgemäßen Verfahren der Feststoffstrom der ersten Trennvorrichtung 4 (FFT4) hauptsächlich in den Reaktor 1 zurückgeführt. Nur ein Feststoffteilstrom wird dem Reaktor 2 zugeführt. Das Fördern feststoffhaltiger Stoffströme ist in der Praxis problematisch, insbesondere dann, wenn ein Feststoffstrom - wie beim PAQUES-Verfahren - in zwei Teilströme getrennt werden muß. Um Betriebsstörungen zu vermeiden, muß dann die Rückführung zum Reaktor 1 getrennt von der Feststoffzufuhr zum Reaktor 2 erfolgen. Zum Ausgleich des Wasserverlustes und zur Gewährleistung eines misch- und pumpfähigen Reaktorinhalts muß Wasser, d.h. Methanreaktorablauf, dem Reaktor 1 zusätzlich zugeführt werden. Diese Maßnahmen erfordern einen im Vergleich zum erfindungsgemäßen Verfahren größeren und komplizierteren maschinentechnischen Aufwand.

Aus Tabelle 1 ist ersichtlich, daß z.B. für die Feststoffhydrolyse bei Bioabfall ein pH-Wert von 6,4 optimal ist. Bezüglich der Stabilität des pH-Wertes in der Feststoffhydrolyse ist jedoch der pH-Wertebereich von 6,0 bis 6,8 relativ kritisch.

**Tabelle 1**

| Versäuerung Reaktor 1 | SRT [d] | Feststoffhydrolyse Reaktor 2 | |
|---|---|---|---|
| pH | | pH | Abbau der zugef. org. Feststoffe [%] |
| 4,5 - 6,0 | 3 | 5,5 | 44 |
| | 3 | 6,4 | 70 |
| | 3 | 6,7 | 38 |
| SRT ("solid retention time") gibt hier die Feststoffverweilzeit an. | | | |

Tabelle 2 ist ein ähnliches Verhalten bei der Hydrolyse von Cellulose in Abhängigkeit vom pH-Wert der Feststoffhydrolyse zu entnehmen.

**Tabelle 2**

| SRT [d] | Feststoffhydrolyse Reaktor 2 | |
|---|---|---|
| | pH | Abbau der zugef. Cellulose [%] |
| 3 | 5,5 | 41 |
| 3 | 6,4 | 44 |
| 3 | 6,7 | 49 |

In Tabelle 3 ist ein Vergleich der verschiedenen Verfahrensführungen zur Biomethanisierung organischer Feststoffe aus Bioabfall bezüglich des Abbaues der organischen Trockenmasse angegeben, der klar die Vorteile des erfindungsgemäßen Verfahrens in Bezug auf einen schnelleren und wesentlich vollständigeren Abbau der zugeführten organischen Trockenmasse belegt.

**Tabelle 3**

| Verfahren | Quelle | Verweilzeit [d] | Abbau der zugef. org. Trockenmasse [%] |
|---|---|---|---|
| einstufig | Rettich | 12 | 42 |
| zweistufig | Rodde | 12 | 50 |
| mehrstufig mit pH-Wert gesteuerter Feststoffhydrolyse | eigene Versuche | 9 | 74 |

Siehe hierzu Rettich, S.: Biogas und Kompost aus Küchen- und Gartenabfällen Pilotversuch in Rottweil in : Thomé-Kozmiensky: Biogas - Anaerobtechnik in der Abfallwirtschaft (EF-Verlag, Berlin 1989) und Rodde, Chr. und W. Westphal: Anaerobe Vergärung als Vorstufe zur Kompostierung, 42. Informationsgespräch 1989, (ANS Info-Band Heft 16, S. 198).

Zur Steuerung dieses kritischen pH-Wertes werden erfindungsgemäß dem ersten Reaktor gelöste und nichtgelöste organische Substanzen direkt entnommen und insbesondere zur Senkung des pH-Wertes dem zweiten Reaktor zugeführt; unabhängig davon kann der pH-Wert im zweiten Reaktor dadurch beeinflußt werden, insbesondere erhöht werden, daß dem zweiten Reaktor gelöste und nichtgelöste organische Substanzen entnommen werden und von diesen zumindest ein Teil der gelösten organischen Substanzen dem dritten Reaktor zugeführt werden, wobei der restliche Teil dem Reaktor 2 wieder zurückgeführt wird.

Durch die Absorption eines Teils des bei der Methanisierung gebildeten Kohlendioxids besitzt der Ablauf des dritten Reaktors eine entsprechende Pufferkapazität, die durch Rückführung in die Hydrolysestufe im zweiten Reaktor zur pH-Wertanhebung nutzbar ist, zur Erhöhung der Pufferkapazität im gesamten System beiträgt und zur Steuerung des Verhältnisses von gelösten zu nichtgelösten Substanzen, somit des Feststoffgehaltes im zweiten Reaktor, nutzbar ist. Hierfür ist vorgesehen, daß vom dritten Reaktor ein feststoffarmer Stoffstrom dem zweiten Reaktor zugeführt wird. Eigene Versuche ergaben eine Säurekapazität dieses Stoffstroms von ca. 180 mval/l. Zum Senken des pH-Wertes von z.B. 6,7 auf 6,3 wurden 24 mval/l benötigt.

Tritt bei einem substratbedingten Absinken der Hydrolyseleistung ein geringer Anstieg des pH-Wertes auf, führt dies in diesem Bereich zu einer starken Zunahme der Methanisierungsleistung der Biozoenose des Hydrolysesektors. Dadurch steigt sprunghaft der Säureverbrauch und der pH-Wert steigt weiter an. Als Folge davon erhöht sich die Methanbildung noch stärker und der Säureverbrauch steigt weiter an.

Aufgrund der verstärkten Methanisierungsleistung und der hohen Pufferkapazität im Hydrolysereaktor sind folglich zum Absenken des pH-Wertes große Mengen an Säuren notwendig. Bringt bei einem zweistufigen System in dieser Situation der aktuelle Zulauf nicht genügend Säure oder leicht vergärbare Substanz mit, kann der pH-Wert nicht mehr stabilisiert werden und driftet in den neutralen Bereich ab. Im neutralen Bereich ist dann der pH-Wert sehr stabil, da die Feststoffhydrolyse der geschwindigkeitsbestimmende Schritt ist und somit sämtliche produzierten Säuren sofort methanisiert werden.

Ein Absenken des pH-Wertes in diesem Prozeßzustand ist nur durch eine Zugabe größerer Mengen Säure möglich. Soll nicht zusätzlich Säure zum System dosiert werden, muß zur pH-Korrektur eine größere Menge weitgehendst versäuerten Substrats zur Verfügung stehen. Dies ist prinzipiell nur bei dem hier beschriebenen dreistufigen System der Fall.

Ist jedoch der Input stark versäuert, so daß für ein zweistufiges System genügend Säure zur pH-Wertsteuerung vorhanden wäre, erfordert die ständig hohe Säurezufuhr zum Hydrolysereaktor auch eine entsprechend hohe Säureabfuhr. Da in diesem Fall die Säurekonzentration im Zulauf höher ist als im Hydrolysereaktor, wird bei direkter Zufuhr des Inputs zum Hydrolysereaktor, wie bei Buchholz, Gijzen und Zwart beschrieben (siehe a.a.O.), die Säure des Inputs zuerst verdünnt. Dies bedeutet, daß im Vergleich zu dem hier beschriebenen dreistufigen System zur Abfuhr der gleichen Säuremenge zur Methanisierung mehr Substrat zur Fest-/Flüssig-Trennung gefördert werden muß. Folglich ist dieses Aggregat größer zu dimensionieren.

Weiterhin ist es bei dem erfindungsgemäßen Verfahren möglich, den Wassergehalt in dem System dadurch zu steuern, daß dem dritten Reaktor überschüssiges Wasser entnommen wird.

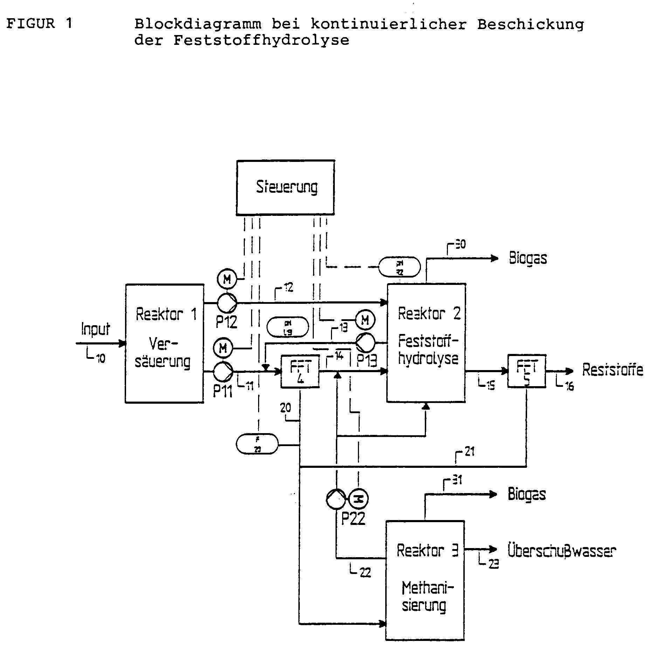

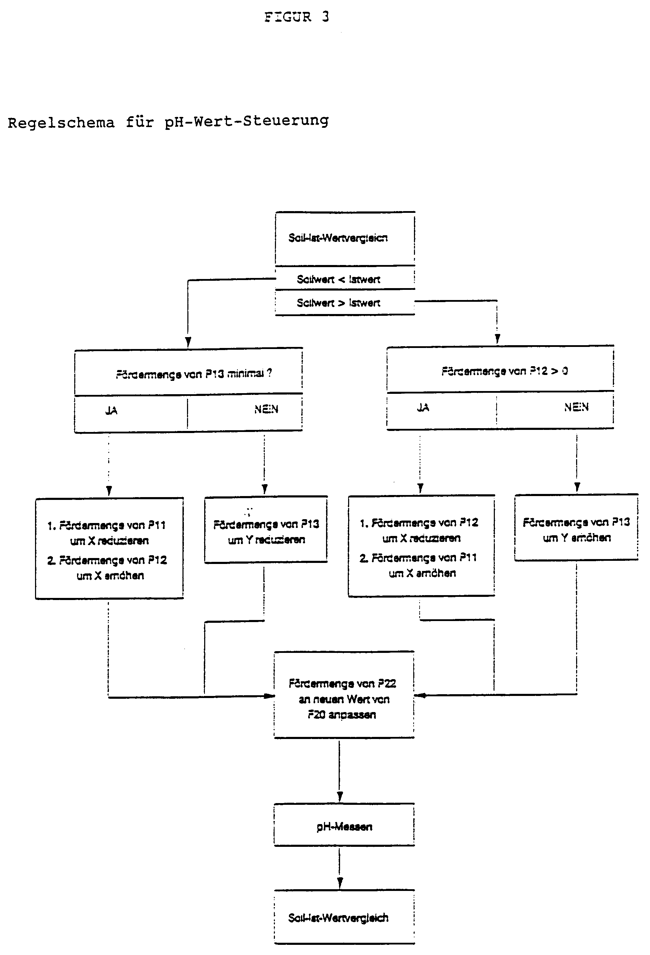

Das erfindungsgemäße Verfahren ermöglicht somit die optimale Einstellung der Bedingungen bei der Feststoffhydrolyse im Reaktor 2, insbesondere die unabhängige Einstellung von pH-Wert, Feststoffkonzentration und Feststoffverweilzeit im Reaktor 2. In Fig. 3 in Verbindung mit Fig. 1 ist das Regelschema für die pH-Wert-Steuerung im einzelnen dargestellt.

Der pH-Wert wird durch Verändern der Förderströme der Stellglieder (z.B. Pumpen) P11, P12 und P13 gesteuert. Am besten erfolgt seine Messung direkt im Reaktor 2 (pH R2). Ist dies aus praktischen Gründen nicht sinnvoll, bietet sich eine pH-Wertmessung in Leitung 13 (pH L13) an. Dann muß jedoch ein bestimmter Rezirkulationsstrom (13) gewährleistet werden. Diese Durchflußgröße legt die minimale Förderleistung von P 13 fest.

Die Feststoffkonzentration im Hydrolysereaktor wird durch ein entsprechendes Verhältnis von rückgeführtem Methanisierungsablauf (22), der über die Fördermenge von P22 gesteuert wird, zur produzierten Flüssigphase (20), die mit dem Durchflußmengenmesser 20 gemessen wird, gesteuert.

Die Feststoffverweilzeit in der Feststoffhydrolyse (Reaktor 2) wird bei vorgegebenem Reaktorvolumen über die zugeführte Menge an versäuertem Gemisch (Summe von 11 und 12) bestimmt.

Zur pH-Wert-Absenkung wird als erste Maßnahme die Rückführung von Reaktorinhalt zur FFT4 (13) reduziert. Dies wird durch Verringern der Fördermenge von P13 erreicht. Gleichzeitig wird die Rückführung von Methanisierungsablauf zum Reaktor 2 (22) entsprechend dem vorgebgebenen Verhältnis ebenfalls verringert. Dadurch kann der Feststoffgehalt im Reaktor 2 konstant gehalten werden und die pH-Wert-Absenkung wird verstärkt.

Ist die Fördermenge von P13 minimal oder Null und eine weitere pH-Wert-Absenkung notwendig, wird versäuertes Gemisch (12) direkt dem Reaktor 2 zugeführt und (11) gesenkt. Dazu wird die Fördermenge von P12 erhöht und die von P11 entsprechend gesenkt. Durch Anpassung der Fördermenge von P22 an die produzierte Flüssigphase (F20) entsprechend dem vorgegebenen Verhältnis wird der Feststoffgehalt im Reaktor 2 konstant gehalten.

Zur pH-Wert-Anhebung wird zuerst (11) erhöht und (12) entsprechend gesenkt. Hierzu wird die Fördermenge von P11 erhöht und die von P12 entsprechend gedrosselt. Damit die Feststoffkonzentration im Reaktor 2 konstant bleibt, wird gleichzeitig die Zufuhr von Methanisierungsablauf entsprechend erhöht. Dadurch wird der pH-Wert-Anstieg zusätzlich begünstigt.

Ist die vollständige Zufuhr des versäuerten Gemisches über (11) nicht ausreichend, wird (13) und (22) erhöht. Dazu wird die Fördermenge von P13 erhöht und die Rückführung des Methanisierungsablaufs an die produzierte Filtratmenge (F20) angepaßt. Die Feststoffkonzentration in Reaktor 2 wird dadurch konstant gehalten.

Die Feststoffkonzentration in Reaktor 2 wird verändert, indem das Verhältnis von rückgeführtem Methanisierungsablauf zu produzierter Flüssigphase angepaßt wird. Anschließend werden dann die Fördermengen von P11, P12 und P13 entsprechend eingestellt, so daß der Soll-pH-Wert in Reaktor 2 gewährleistet ist.

Bei den beschriebenen Eingriffen zur Steuerung von pH-Wert und Feststoffkonzentration in Reaktor 2 kann die zugeführte Menge an versäuertem Gemisch (Summe von 11 und 12) konstant gehalten werden. Dadurch wird die Feststoffverweilzeit in Reaktor 2 nicht verändert.

Eine derartige Steuerung des pH-Wertes unabhängig von der Feststoffkonzentration und der Feststoffverweilzeit in Reaktor 2 wird durch das PAQUES-Verfahren nicht ermöglicht.

Ein Senken des pH-Werts im RUDAD bei PAQUES ist nur durch eine der folgenden drei Maßnahmen möglich:
1. Erhöhung der Feststoffzufuhr (Belastungssteigerung)
2. Reduzierung der Rückführung an Methanisierungsablauf
3. Veränderung der Trennleisung der Fest/Flüssig-Trennung
Jede der drei Maßnahmen führt jedoch dazu, daß für die Feststoffhydrolyse ungünstigere Bedingungen entstehen.

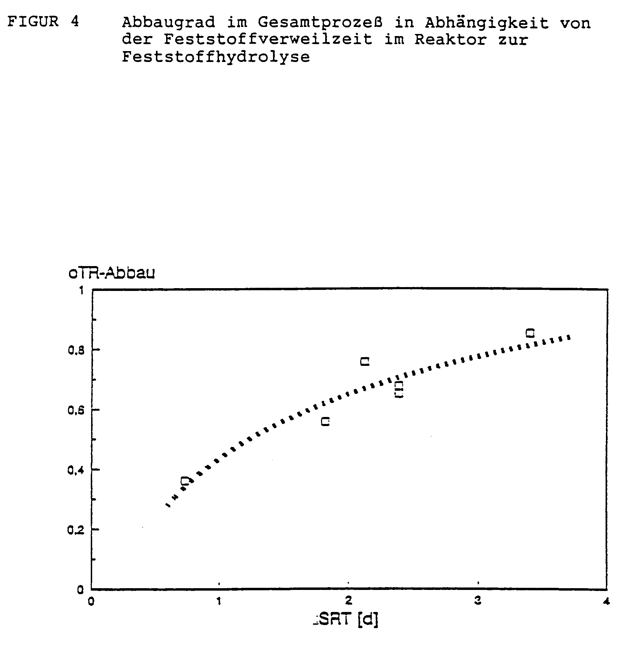

Durch eine Erhöhung der Feststoffzufuhr muß zum Konstanthalten der Feststoffkonzentration auch die Zufuhr an Methanisierungsablauf erhöht werden. Dies führt zu einer verstärkten Reduzierung der Verweilzeit im Reaktor 2. Kürzere Feststoffverweilzeiten führen zu einem geringeren Abbaugrad (Fig.4). Zusätzlich wirkt die Erhöhung der rückgeführten Menge an Methanisierungsablauf einem pH-Wert-Abfall entgegen.

Wird die Rückführung des Methanisierungsablaufs reduziert und die Feststoffzufuhr konstant gehalten oder erhöht, kann durch Reduzieren der Rezirkulation im RUDAD-Reaktor ein Feststoffanstieg verhindert werden. Ist die Rezirkulation auf Null und muß zur pH-Wert-Absenkung die Rückführung von Methanisierungsablauf noch weiter reduziert werden, steigt die Feststoffkonzentration im Reaktor 2. Dies kann zur Hemmung der Feststoffhydrolyse durch Transportlimitierung oder zur Überlastung und Beschädigung der Maschinentechnik führen.

Eine gezielte Veränderung der Trennleistung der Fest/Flüssig-Trennung ist maschinentechnisch aufwendig. Hinzu kommt, daß aufgrund des sich häufig verändernden Trennverhaltens der zu verarbeitenden Abfallgemische eine häufige Bestmmung der Trennleistung der Fest/Flüssig-Trennung notwendig ist. Da für die vorliegenden Stoffströme aufgrund ihrer Zusammensetzung keine on-line-Bestimmung der Trennleistung möglich ist, muß diese durch Laboranalysen ermittelt werdcn. Somit ist mit diesem Parameter keine zeitnahe Umsetzung der Steuerung möglich.

Zur pH-Wert-Anhebung im Reaktor 2 stehen zwei Reaktionsmöglichkeiten zur Verfügung, die ebenfalls ungünstige Auswirkungen haben:
1. Erhöhung der Rückführung an Methanisierungsablauf
2. Reduzierung der Feststoffzufuhr
Die Erhöhung der Rückführrate an Methanisierungsablauf führt bei konstanter Feststoffzufuhr und gleichzeitiger Erhöhung der Rezirkulation im RUDAD-Reaktor zu keiner Beeinflußung der Feststoffkonzentration und Feststoffverweilzeit.

Eine Reduzierung der Feststoffzufuhr hat ein Absinken der Durchsatzleistung des Prozesses zur Folge.

Aufgrund der dargelegten bestehenden Verknüpfungen im PAQUES-Verfahren ist es offensichtlich, daß mit diesem Verfahren im Reaktor 2 (RUDAD) die Paramter pH-Wert, Feststoffkonzentration und Feststoffverweilzeit nicht unabhängig voneinander zu steuern sind. Wie dargelegt ist jedoch eine definierte Einstellung aller drei Parameter für die Geschwindigkeit der Feststoffhydrolyse entscheidend.

Dagegen ist das erfindungsgemäße Verfahren dem Stand der Technik bezüglich Steuerung des pH-Werts und der Feststoffkonzentration im Reaktor 2 überlegen. Mit diesem Steuerungskonzept können pH-Wert, Feststoffkonzentration und Feststoffverweilzeit unabhängig voneinander eingestellt werden.

Zusammenfassend ergeben sich erfindungsgemäß folgende Parameter und Steuergrößen bei der Feststoffhydrolyse:

| Parameter | Steuergröße |
|---|---|
| Feststoffverweilzeit | Zuzuführende Fracht an versäuertem Gemisch (Summe von 11 und 12) + Abfuhr |
| Feststoffkonzentration in der Feststoffhydrolyse | Verhältnis von rückzuführendem Methanisierungsablauf (22) zu produzierter Flüssigphase (20) + Zufuhr 11 + 12 |
| pH-Wert in der Feststoffhydrolyse | Fördermengen der Stellglieder P11, P12 und P13 |

Ein Vergleich prozeßrelevanter Parameter des PAQUES-Verfahrens gemäß dem Stand der Technik und des erfindungsgemäßen Verfahrens stellt sich wie folgt dar:

| prozeßrelevante Parameter | PAQUES | erfindungsgemäßes Verfahren |
|---|---|---|
| A. pH-Wert-Steuerung | beeinflußt B. und C. | unabhängig von B. und C. |
| B. Feststoffkonzentration in Reaktor 2 | wird von A.+C. beeinflußt | kann unabhängig von A. und C. konstant gehalten werden |
| C. Feststoffverweilzeit in Reaktor 2 | wird von der Feststoffbeschickung festgelegt | wird von der Feststoffbeschickung festgelegt |

Im RUDAD-Reaktor spielen für den Feststoffabbau Ciliaten und anaerobe Pilze eine entscheidende Rolle (Gijzen, H.J. et al: Anaerobic digestion of cellulose fraction of domestic refuse by means of rumen mikroorganisms, Biotechnology and Bioengineering 32 (1988), S. 749-755). Da diese meistens nicht in den zu verarbeitenden Feststoffen vorhanden sind, erfordert dies das Einfahren des Reaktors 2 mit einem speziellen Impfschlamm. Das erfindungsgemäße Verfahren erfordert dies nicht. Im Reaktor 2 werden aus den in den zugeführten Feststoffen enthaltenen Mikroorganismen geeignete selektiert. Dies erfolgt durch entsprechende Einstellung des pH-Werts und der Feststoffverweilzeit (Größe der Generationszeit der gewünschten Mikroorganismen).

Die Ausbeute der Biogas-, insbesondere Methangasgewinnung läßt sich dadurch erhöhen, daß im zweiten Reaktor zusätzlich zur Feststoffhydrolyse eine Methanisierung vorgenommen wird und dem zweiten und dem dritten Reaktor Biogas, insbesondere Methangas, entnommen wird. Dadurch kann die Raumbelastung des dritten Reaktors gesenkt und damit der Abbaugrad im dritten Reaktor erhöht werden.

In der nachfolgenden Tabelle 4 ist die Gaszusammensetzung der Feststoffhydrolyse und der Methanisierung in Vol.-% aufgeführt.

**Tabelle 4**

| | pH-Wert | CO₂ | CH₄ |
|---|---|---|---|
| Feststoffhydrolyse | 5,5 - 7,7 | 25 - 80 | 19 - 74 |
| | 5,9 | | 20 |
| | 6,4 | | 26 |
| | 7,0 | | 47 |
| | 7,4 | | 59 |
| Methanisierung | - | 19 - 33 | 66 - 80 |

Die Vorteile dieses erfindungsgemäßen Verfahrens werden durch Verwendung der in den Ansprüchen 29 bis 37 beschriebenen Vorrichtung nutzbar.

Die Erfindung wird nachstehend anhand der beigefügten Figuren im einzelnen beschrieben.

Es zeigen :
- Figur 1: ein Blockdiagramm einer Vorrichtung für die Durchführung des Verfahrens bei kontinuierlicher Beschickung der Feststoffhydrolysestufe, mit pH-Wert-Steuerung, und
- Figur 2: ein Blockdiagramm einer Vorrichtung zur Durchführung des Verfahrens bei diskontinuierlicher Beschickung der Feststoffhydrolysestufe, mit pH-Wert-Steuerung,
- Figur 3: ein Regelschema für die pH-Wert-Steuerung gemäß der Erfindung,
- Figur 4: den Abbaugrad im Gesamtprozeß in Abhängigkeit von der Feststoffverweilzeit im Reaktor zur Feststoffhydrolyse,

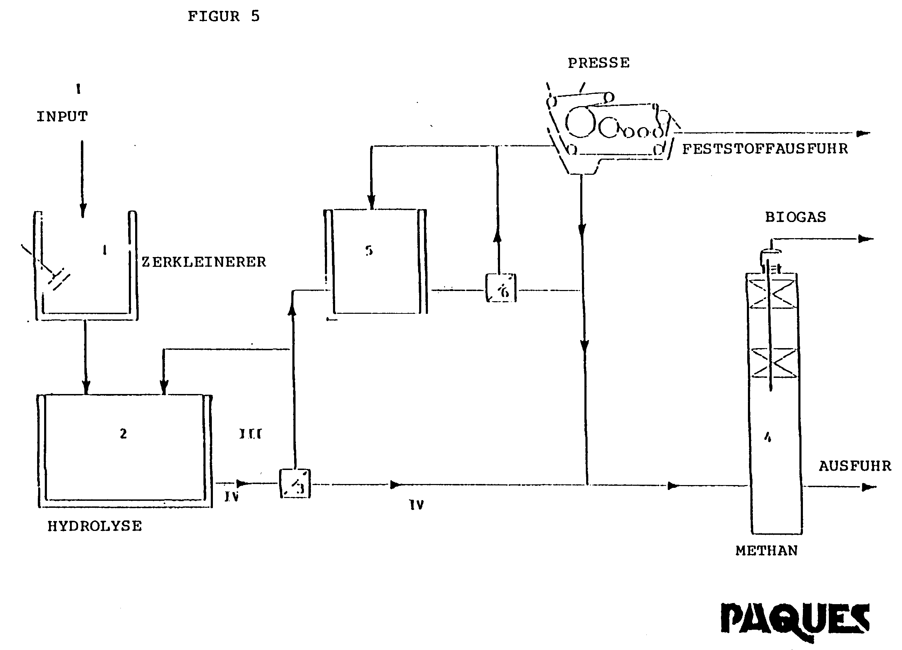

- Figur 5: das PAQUES-Verfahren gemäß dem Stand der Technik.
- Figur 6: zeigt ein Blockdiagramm der erfindungemäßen Vorrichtung bei vereinfachter Verfahrensführung,
- Figur 7: zeigt ein Regelschema für die pH-Wert-Steuerung bei vereinfachter Verfahrensführung.

Nachstehend wird zunächst die Vorrichtung im einzelnen beschrieben.

Im wesentlichen weist die Vorrichtung einen ersten Reaktor 1 für die Versäuerung der organischen Substanzen, einen zweiten Reaktor 2 für zumindest die Feststoffhydrolyse der angesäuerten nichtgelösten organischen Substanzen und einen Reaktor 3 für die Methanisierung der gelösten organischen Substanzen auf.

Am Reaktor 1 ist ein Einlaß bzw. Input 10 angebracht, über welchen dieser mit den zu bearbeitenden organischen Substanzen beschickbar ist.

Mit einem kommunizierenden Element 11, welches aus einer Leitung, einem Kanal oder ähnlichem bestehen kann, ist der Reaktor 1 mit einer Trennvorrichtung 4 verbunden, in der in geeigneter und an sich bekannter Weise gelöste und nichtgelöste Stoffe trennbar sind und dabei die gelösten Stoffe als Flüssigfraktion über ein kommunizierendes Element 20 abführbar sind und die nichtgelösten Stoffe über ein kommunizierendes Element 14 abführbar sind.

Das Element 14 ist mit dem Reaktor 2 verbunden, das Element 20 mit dem Reaktor 3, so daß dem Reaktor 1 entnommene gelöste Substanzen dem Reaktor 3 zuführbar sind und dem Reaktor 1 entnommene nichtgelöste dem Reaktor 2 zuführbar sind.

Über ein weiteres kommunizierendes Element 12 ist der Reaktor 1 direkt mit dem Reaktor 2 verbunden, wobei dadurch die im Reaktor 1 befindlichen Substanzen dem Reaktor 2 direkt zuführbar sind. Mit einem kommunizierenden Element 13 ist der Reaktor 2 mit dem Element 11 verbunden, hierdurch sind gelöste und nichtgelöste Substanzen dem Reaktor 2 entnehmbar, der Trennvorrichtung 4 zuführbar, wobei die nichtgelösten organischen Substanzen dem Reaktor 2 zurückführbar sind, und die gelösten organischen Substanzen dem Reaktor 3 zuführbar sind. Im Gegensatz zur Vorrichtung zur Durchführung des PAQUES-Verfahrens weist die erfindungsgemäße Vorrichtung keine Leitung zur Rückführung der Feststoffe in den Reaktor 1 auf.

Der Reaktor 3 ist über ein weiteres kommunizierendes Element 22 mit dem Reaktor 2 direkt verbunden oder alternativ zusätzlich mit dem Element 14 verbunden, wodurch im Reaktor 3 befindliche Substanzen dem Reaktor 2 zuführbar sind.

Der Reaktor 3 weist einen für die gesteuerte Entnahme von Wasser geeigneten Auslaß 23 auf.

Zur Steuerung des pH-Wertes im Reaktor (2) sind die Stellglieder P11, P12 und/oder P13 vorgesehen.

An dem Reaktor 3 sowie dem Reaktor 2 sind Auslässe (31 bzw. 30) für die Entnahme von Gas, insbesondere Biogas wie z.B. Methangas, angebracht, welche geeignete Anschlüsse für weitere für die Entnahme anzubringende Leitungen aufweisen.

In einem ersten Ausführungsbeispiel, welches vorzugsweise bei kontinuierlicher Beschickung der Feststoffhydrolyse verwendet wird, ist der Reaktor 2 über ein kommunizierendes Element 15 mit einer Trennvorrichtung 5 (FFT 5) für die Trennung gelöster und nichtgelöster Stoffe in eine Feststofffraktion und eine Flüssigfraktion verbunden. Die Trennvorrichtung ist mit einem weiteren kommunizierenden Element 21 mit dem Element 20 verbunden, so daß dem Reaktor 2 entnommene gelöste Stoffe dem Reaktor 3 zuführbar sind.

Über einen an der Trennvorrichtung 5 angebrachten Auslaß 16 oder ein kommunizierendes Element 16 sind der Trennvorrichtung 5 Reststoffe entnehmbar.

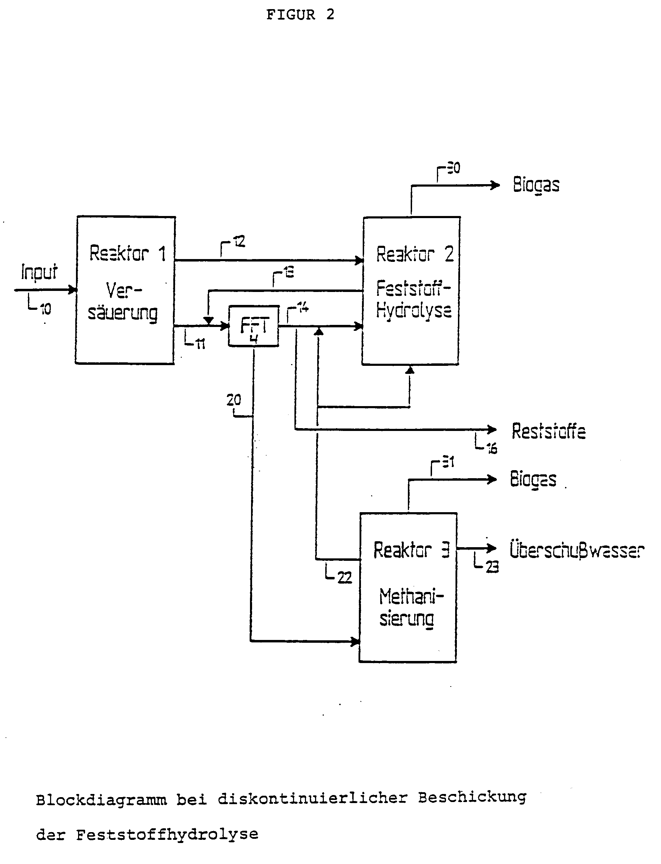

In einem zweiten Ausführungsbeispiel, welches vorzugsweise bei diskontinuierlicher Beschickung der Feststoffhydrolyse Verwendung findet, ist das Element 14 mit einem kommunizierenden Element 16 verbunden, so daß die durch die Trennvorrichtung 4 gewonnene Feststofffraktion über das Element 16 als Reststoff entnehmbar ist. Die pH-Wert-Steuerung bei diskontinuierlicher Beschickung ist im einzelnen der Figur 2 entnehmbar.

Nachstehend wird das Verfahren im einzelnen beschrieben.

Über den Input 10 werden dem ersten Reaktor 1 gelöste und/oder nichtgelöste organische Substanzen zugeführt und einer Versäuerung unterzogen. Die zumindest angesäuerten organischen Substanzen werden dem Reaktor 1 entnommen und über das Element 11 einer Trennvorrichtung zugeführt, in welcher diese in eine Feststofffraktion, die im wesentlichen die nichtgelösten organischen Substanzen enthält, und in eine Flüssigfraktion, die im wesentlichen die gelösten organischen Substanzen enthält, getrennt werden.

Die Flüssigfraktion wird über das Element 20 dem Reaktor 3 zugeleitet, die Feststofffraktion über das Element 14 dem Raktor 2.

Im Reaktor 2 werden die nichtgelösten organischen Substanzen
einer Feststoffhydrolyse unterzogen. Zusätzlich können die bei der Feststoffhydrolyse gebildeten gelösten organischen Substanzen einer Methanisierung unterzogen werden.

In Reaktor 3 findet eine Methanisierung der darin befindlichen organischen Substanzen statt.

In weiterer Ausgestaltung des Verfahrens werden dem Reaktor 1 über das Element 12 organische Substanzen entnommen und dem Reaktor 2 für dessen pH-Wert-Steuerung, insbesondere -Senkung, direkt zugeführt.

Über das Element 13 werden in weiterer Ausgestaltung der Erfindung dem Reaktor 2 organische Substanzen entnommen und über einen Teil des Elementes 11 der Trennvorrichtung 4 zugeführt, durch welche die daraus gewonnene Flüssigfraktion über das Element 20 dem Reaktor 3 zugeleitet und die mittels der Trennvorrichtung gewonnene Feststofffraktion über das Element 14 dem Reaktor 2 zugeleitet wird. Hierdurch kommt es durch das Abscheiden der gelösten organischen Substanzen in der Regel zu einer pH-Wert-Erhöhung im Reaktor 2, welche für die Steuerung des pH-Wertes des Reaktors 2 verwendet wird.

Der Feststoffgehalt im Reaktor 2 wird dadurch gesteuert, daß vom Reaktor 3 ein feststoffarmer Stoffstrom über das Element 22 dem Reaktor 2 zugeführt wird. Darüber hinaus wird dabei die Pufferkapazität der im Reaktor 2 vorhandenen Substanzen erhöht.

Durch die Entnahme von überschüssigem Wasser aus Reaktor 3 über das kommunizierende Element 23 kann das Verhältnis des dem ersten Reaktor 1 mit den organischen Substanzen zugeführten Wassers zu dem dem zweiten und dem dritten Reaktor entnommenen Wasser kompensiert werden, so daß damit der Wassergehalt des Gesamtsystems, insbesondere der Reaktoren 2 und 3, gesteuert werden kann.

In einem ersten Ausführungsbeispiel wird der Reaktor 2 kontinuierlich beschickt und diesem kontinuierlich über das Element 15 organische Substanzen entnommen, welche in der Trennvorrichtung 5 in eine Feststofffraktion und eine Flüssigfraktion getrennt werden.

Die Feststofffraktion wird der Trennvorrichtung 5 über das Element 16 als Reststoff entnommen. Die Flüssigfraktion wird dem Reaktor 3 über das Element 21 und ein Teil des Elementes 20 zugeführt.

In einem zweiten Ausführungsbeispiel wird der Reaktor 2 diskontinuierlich beschickt; hierbei findet die ebenfalls diskontinuierliche Reststoffentnahme über ein mit dem Element 14 verbundenes weiteres Element 16 statt.

Die bei dem Verfahren gewonnenen Biogase werden in beiden Ausführungsbeispielen dem Reaktor 2 und dem Reaktor 3 über die Auslässe 30, 31 entnommen.

Im folgenden wird nochmals ein Überblick über die wichtigsten Schritte des erfindungsgemäßen Verfahrens gegeben.

Mit dem erfindungsgemäßen Verfahren wird die anaerobe biologische Hydrolyse und Biomethanisierung von komplexen organischen Stoffen, wie z.B. von Biopolymeren, verbessert.

Die Hemmung der Feststoffhydrolyse in der Hydrolysestufe bei kontinuierlicher Zufuhr komplexer Substrate mit leicht vergärbaren Bestandteilen wird erfindungsgemäß durch eine vorgeschaltete Verfahrensstufe unterbunden (Versäuerungsstufe). Dadurch werden vermehrt organische Feststoffe abgebaut. Dies führt erstens zu einem höheren Biogasertrag und zweitens durch die geringere Produktion an festen Reststoffen zu niedrigeren Entsorgungskosten.

Durch die Steuerung des pH-Wertes und der Feststoffkonzentration im Hydrolysereaktor wird die biologische Hydrolyse der Feststoffe beschleunigt. Dies führt bei gleichen Verweilzeiten zu einem höheren Abbau, bzw. bei gleichem Abbaugrad wegen der kürzeren Verweilzeiten zu kleineren Reaktorgrößen.

Ferner kann bei einer gleichbleibenden, kontinuierlichen Beschikkung des Systems im Hydrolysereaktor der optimale pH-Wert eingestellt werden. Somit ist zur pH-Wert-Steuerung keine Veränderung der Beschickungsmenge notwendig.

Häufig ist bei der Biomethanisierung fester Stoffe eine pH-Wert-Steuerung wegen des zusätzlichen Bedarfs an Säuren oder Laugen nicht betriebswirtschaftlich. In diesem Verfahren werden zur pH-Wert-Steuerung ausschließlich im Prozeß entstehende Produkte verwandt. Dadurch wird die Betriebswirtschaftlichkeit erhöht.

Bei Verzicht auf Laugendosierung wird ein Absinken des pH-Werts in den biologischen Reaktoren üblicherweise durch Reduzierung der Reaktorbelastung korrigiert. Dadurch sinkt die Durchsatzleistung des Verfahrens. Um eine minimale Durchsatzleistung einhalten zu können, muß deshalb die biologische Stufe überdimensioniert werden. Durch die pH-Wert-Steuerung kann einem pH-Wert-Abfall ohne Reduzierung der Durchsatzleistung begegnet werden, und somit können diesbezüglich die Auslegungsreserven eingespart werden.

Das Verfahren besteht aus getrennten Reaktoren zur Versäuerung, Hydrolyse und Methanisierung. Die bei der Versäuerung und Hydrolyse gebildeten organischen Säuren werden mit einer Fest/Flüssig-Trennung abgetrennt und dem Methanreaktor zugeführt. Der Ablauf des Hydrolysereaktors wird entwässert, die Flüssigphase dem Methanreaktor zugeführt und die Feststoffe ausgeschleust. Ein Teil des Methanreaktorablaufs wird in den Hydrolysereaktor zurückgeführt.

Die organischen Stoffe liegen in wäßriger Phase mit einem Trockenrückstand von 1 bis 25 Gew.-% als ein Gemisch von gelösten und ungelösten Sustanzen vor. Das Gemisch (10) wird dem Reaktor 1 zugeführt. Dieser Behälter dient der Speicherung und, sofern die gelösten organischen Substanzen nicht bereits spontan versäuert sind, zur weitgehenden Fermentation der anaerob abbaubaren gelösten Substanzen. Werden mit dem Substratgemisch nicht ausreichend fermentative Mikroorganismen in den Versäuerungsreaktor eingetragen, so kann dieser mit geeigneten Bakterien angeimpft und in einer geeigneten Weise betrieben werden, so daß diese sich ausreichend vermehren und nicht ausgewaschen werden. Wegen der weitgehenden Fermentation der gelösten Bestandteile des Inputs stellt sich in Reaktor 1 ein niedriger pH-Wert ein.

Aus Reaktor 1 wird das versäuerte Gemisch in den Reaktor 2 zur Hydrolyse der Feststoffe gegeben. Da, wie z.B. bei Bioabfall, sein pH-Wert zu niedrig ist (siehe Tabelle 1), wird zur Anhebung des pH-Werts im Reaktor 2 auf ein zur Feststoffhydrolyse optimales Niveau das Gemisch über eine Fest/Flüssig-Trennung (FFT4) entwässert (11). Dadurch werden durch Fermentation gebildete Säuren mit der Flüssigphase (20) zum Reaktor 3 abgeführt. Die entwässerte Feststofffraktion (14) wird dem Reaktor 2 zugeführt. Durch Rückführung des Ablaufs aus Reaktor 3 (22) wird dem Hydrolysereaktor Pufferkapazität zugeführt. Die Rückführung des Methanisierungsablaufs kann entweder direkt oder zusammen mit der Feststofffraktion (14) erfolgen.

Durch die Rückführung des Methanisierungsablaufs werden ständig methanogene Mikroorganismen in den Reaktor zur Feststoffhydrolyse eingetragen. Dadurch siedelt sich im Reaktor 2 neben der hydrolytischen auch eine methanogene Biozoenose an, die bei der Feststoffhydrolyse gebildete Säuren direkt zu Biogas (30) umwandelt. Ihre Aktivität wird maßgeblich durch den pH-Wert der Hydrolysestufe bestimmt. Entspricht der Abbau der gebildeten Säuren durch die methanogenen Mikroorganismen der Produktionsrate an Säuren durch die hydrolytischen Mikroorganismen, so bleibt im Reaktor der pH-Wert konstant.

Steigt bei dieser Verfahrensführung durch eine zu hohe Aktivität der methanogenen Mikroorganismen der pH-Wert im Reaktor 2 für eine optimale Feststoffhydrolyse zu stark an, kann durch die direkte Zugabe des versäuerten Gemisches (12) der pH-Wert wieder auf einen optimalen Wert gesenkt werden.

Sinkt bei vollständiger Zufuhr des versäuerten Gemisches aus Reaktor 1 zu FFT 4 dagegen der pH-wert in Reaktor 2 zu stark, können durch eine Rezirkulation des Inhalts von Reaktor 2 (13) gebildete Säuren abgeführt werden. Das Ersetzen der abgeführten Flüssigphase durch Methanisierungsablauf (22) erhöht die Pufferkapazität und begünstigt den direkten Abbau der gebildeten Säuren in der Feststoffhydrolyse (Reaktor 2).

Der Austrag aus der Feststoffhydrolyse (15) erfolgt in Abhängikeit von der notwendigen Feststoffverveilzeit in Reaktor 2. Er wird der FFT 5 zugeführt und entwässert. Die Feststoffe (16) werden ausgeschleust und die Flüssigphase (21) wird der Methanisierung (Reaktor 3) zugeführt.

In der Methanisierung werden die mit den Flüssigphasen aus der Fest/Flüssig-Trennung (20 und 21) zugeführten gelösten Substanzen in Biogas (31) umgewandelt. Ein Teil des produzierten Kohlendioxids wird dabei in der Flüssigphase absorbiert. Dadurch hat der Methanisierungsablauf eine erhebliche Karbonatpufferkapazität. Diese wird zur pH-Wertanhebung in der Feststoffhydrolyse ausgenutzt.

Wird mit dem Input (10) mehr Wasser eingetragen als mit den Strömen Reststoffe (16) und Biogas (30 und 31) ausgetragen wird, ist ein Teil des Methanisierungsablaufs als Überschußwasser (23) auszuschleusen. Ist dagegen der Wassergehalt des Inputs zu niedrig, muß dieser entsprechend erhöht werden, um die Wasserverluste mit den Strömen 16, 30 und 31 zu ersetzen.

Über das Verhältnis von rückgeführtem Methanisierungsablauf (22) zu abgeführter Flüssigphase (20) kann die Feststoffkonzentration in der Feststoffhydrolyse gesteuert werden. Wird für das zu behandelnde Gemisch im Hydrolysereaktor die jeweils optimale Feststoffkonzentration eingestellt, führt dies zur Erhöhung der Reaktionsgeschwindigkeit. Folglich kann die Verweilzeit und somit das Reaktorvolumen der Hydrolysestufe reduziert werden.

Verläuft die Versäuerung der leicht vergärbaren Substanzen in der Versäuerungsstufe nicht vollständig, wird ein Teil dieser Substanzen mit dem Feststoffstrom (14) in die Feststoffhydrolyse eingetragen. Dadurch kann die Hydrolyse der Feststoffe in Reaktor 2 gehemmt werden. Dieser kann durch eine diskontinuierliche Beschickung der Hydrolysestufe begegnet werden. Dabei müssen die Beschickungspausen so lang gewählt werden, daß in dieser Zeit die in Reaktor 2 eingetragenen leicht vergärbaren Substanzen weitgehend versäuert sind und danach noch genügend Zeit für eine weitgehende Hydrolyse der Feststoffe zur Verfügung steht.

Durch eine diskontinuierliche Beschickung der Feststoffhydrolyse (Reaktor 2) aus der Versäuerung (Reaktor 1) kann das zweite Aggregat zur Fest/Flüssig-Trennung (FFT 5) eingespart werden. In diesem Fall erfolgt der Austrag der Feststoffhydrolyse über FFT 4.

In diesem Fall dient der Reaktor 1 außer der Versäuerung auch der Speicherung des Inputs. Der diskontinuierliche Betrieb der Feststoffhydrolyse erfolgt in drei Phasen.

In Phase 1 wird die Feststoffhydrolyse mit einer vorgegebenen Menge an versäuertem Gemisch (11,12) beschickt. Die Verteilung auf 11 oder 12 erfolgt dabei gemäß dem im Reaktor 2 einzustellenden pH-Wert. Entsprechend einem vorgegebenen Verhältnis wird ein Teil der zur Methanisierung abgeführten Flüssigphase 20 durch Rückführung des Methanisierungsablaufs ersetzt.

Steigt der pH-Wert über den Sollwert, wird der Anteil der direkt zugeführten Menge an versäuertem Gemisch (12) erhöht. Dadurch sinkt der pH-Wert im Reaktor 2.

Wird das ganze versäuerte Gemisch über die FFT dem Reaktor 2 zugeführt und der pH-Wert in der Feststoffhydrolyse ist zu niedrig, wird in einer Phase 2 Feststoffhydrolyseinhalt über die FFT rezirkuliert und ein Teil der produzierten Flüssigphase (20) durch Methanisierungsablauf ersetzt. Dadurch erfolgt die Anhebung des pH-Werts in Reaktor 2 auf den vorgegebenen Sollwert. Phase 2 kann entweder anschließend oder zeitgleich zu Phase 1 ablaufen.

Der Austrag aus Reaktor 2 erfolgt in Phase 3, die sich zeitlich an die Phasen 1 und 2 anschließt. Dabei werden die entwässerten Feststoffe nicht wie in Phase 2 in die Feststoffhydrolyse zurückgeführt, sondern aus dem Prozeß ausgeschleust.

Wie entscheidend der pH-Wert des Reaktors 2 das Ergebnis der Feststoffhydrolyse beeinflußt, ist aus Tabelle 1 ersichtlich. Diese Versuchsergebnisse wurden bei der Verwertung von Bioabfall aus der getrennten Sammlung von Siedlungsabfällen in einer halbtechnischen Pilotanlage ermittelt, mit der die hier beschriebene Verfahrensführung realisiert wurde. PH-Wert-Änderungen um 0,3 Einheiten führen schon zu einer deutlichen Reduzierung der Feststoffhydrolyse.

In Reaktor 1 können diese Bedingungen wegen der unkontrollierten Versäuerung nicht eingehalten werden. Der pH-Wert liegt deutlich unter dem Optimum der Feststoffhydrolyse, und bei Schwankungen um 1,5 Einheiten ist die erforderliche pH-Wert-Konstanz nicht möglich.

Der optimale pH-Wert der Feststoffhydrolyse wird maßgeblich von der Zusammensetzung der zu hydrolysierenden Substanzen bestimmt. Liegt für das Gemisch "Bioabfall" das pH-Optimum bei 6,4 (Tabelle 1), wurde die Cellulosefraktion des Bioabfalls bei einem pH-Wert von 6,9 besser hydrolysiert (Tabelle 2).

Vergleicht man die mit der dargestellten Verfahrensführung bei der Verwertung von Bioabfall erzielten Ergebnisse (Tabelle 3) mit in der Literatur berichteten Leistungen zur anaeroben Vergärung von Bioabfall, verdeutlicht sich die Überlegenheit des erfindungsgemäßen mehrstufigen Verfahrens mit pH-Wert gesteuerter Feststoffhydrolyse. Bei kürzerer Verweilzeit wird eine höhere Abbaurate erreicht. Da in der Literatur die Bezüge der Verweilzeiten unterschiedlich sind, wurden für Tabelle 3 die angegebenen Verweilzeiten auf eine einheitliche Bezugsgröße umgerechnet.

Die maschinentechnische Ausführung des Verfahrens ist den spezifischen Eigenschaften der zu behandelnden Substrate anzupassen.

Der Reaktor 1 kann entsprechend der Rheologie, des Versäuerungsgrads und der Kinetik der Versäuerung des Inputs (10) als vollständig durchmischter Reaktor oder einfacher Behälter ausgeführt werden. Reaktor 2 ist entweder als durchmischter oder Kaskade von mehreren Reaktoren oder als plug-flow Reaktor auszulegen. Als Reaktor 3 wurden verschiedene Reaktortypen getestet. Dabei erbrachten Festbettreaktoren bessere Ergebnisse als UASB und Kontaktschlammreaktoren. Auch eine Kaskade von mehreren Reaktoren ist möglich. Reaktor 2 und Reaktor 3 sind beide gasdicht ausgeführt, um eine quantitative Erfassung des produzierten Biogases sicherzustellen. In Tabelle 4 ist die Gaszusammensetzung der beiden Reaktoren angegeben. Der Methangehalt im Reaktor 2 wird stark vom pH-Wert beeinflußt. Je niedriger der pH-Wert um so geringer ist der Methangehalt.

Erfindungsgemäß ist somit auch an der Stelle je eines einzelnen Hydrolyse- bzw. Methanreaktors eine Kaskade, d.h. mehrere solcher Hydrolyse- bzw. Methanreaktoren bevorzugt einsetzbar.

Alle drei Reaktoren können im mesophilen als auch im thermophilen Temperaturbereich betrieben werden. Für Reaktor 1 ist auch der psychrophile Temperaturbereich möglich.

Je nach Korngrößenverteilung und Trennverhalten der Feststoffe können zur Fest/Flüssig-Trennung von 11, 13 und 15 Siebe, Filter, Filterpressen, Schneckenpressen, Zentrifugen oder Dekanter eingesetzt werden.

Die Messung des pH-Werts der Feststoffhydrolyse erfolgt entweder im Reaktor 2 oder in der Leitung zur FFT 4 (14). Aufgrund der besseren Zugänglichkeit ist die Leitung dem Reaktor vorzuziehen. Die Dosierung des versäuerten Gemischs (11,12) in den Reaktor 2 und zur FFT 4 erfolgt mittels Pumpen. Die Förderleistungen dieser Pumpen werden in Abhängigkeit des pH-Werts in Reaktor 2 geregelt. Soll sein pH-Wert gesenkt werden, ist der Förderstrom in 11 zu reduzieren, und der Förderstrom in 12 um den entsprechenden Betrag zu erhöhen. Zur Anhebung des pH-Werts im Reaktor 2 wird als erste Maßnahme die Fördermenge in 11 erhöht und entsprechend in 12 gesenkt. Wird das versäuerte Gemisch vollständig der FFT 4 zugeführt und der pH-Wert im Reaktor 2 ist noch zu tief, wird der Förderstrom in 13 erhöht.

Die Feststoffphase der FFT 4 gelangt entweder durch Gravitation (hierzu muß FFT 4 oberhalb des Reaktors 2 angeordnet sein) oder mittels eines Förderaggregats, z.B. Feststoffpumpe, in die Feststoffhydrolyse. Zur Entschärfung dieses Förderproblems kann der zu rezirkulierende Methanisierungsablauf (22) direkt der Feststoffphase zugemischt und anschließend in Reaktor 2 gefördert werden.

Der Feststoffgehalt im Reaktor 2 wird durch ein entsprechendes Volumenverhältnis von zurückgeführtem Methanisierungsablauf (22) zu abgeführter Flüssigphase (20) eingestellt. Mittels Durchflußmessung in 20 wird das abgeführte Volumen bestimmt und entsprechend dem empirisch ermittelten Volumenverhältnis der Förderstrom in 22 eingestellt.

Es ist eine weiter Aufgabe der Erfindung, eine vereinfachte Ausführungsform der oben beschriebenen Erfindung bereitzustellen. Diese Aufgabe wird durch das in Anspruch 15 näher gekennzeichnete Verfahren und die in Anspruch 23 näher gekennzeichnete Vorrichtung gelöst.

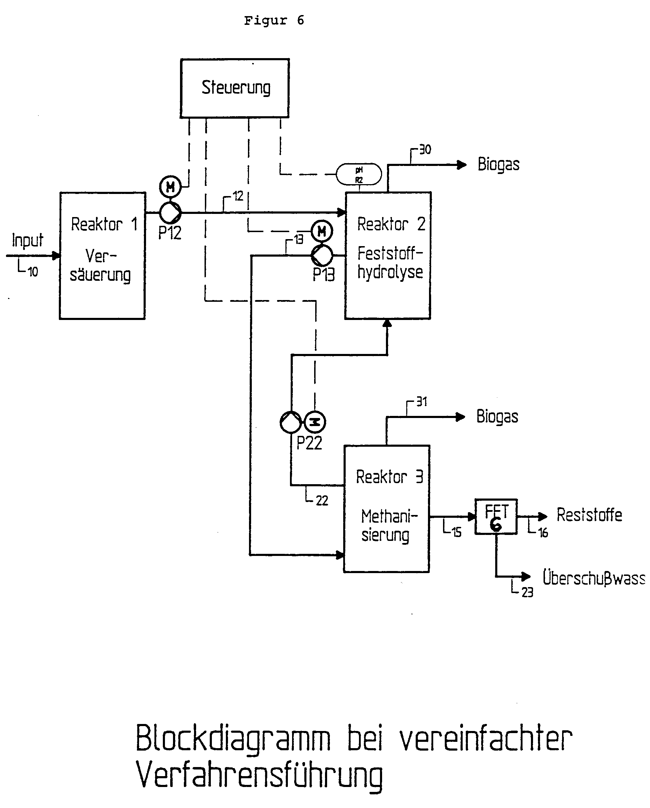

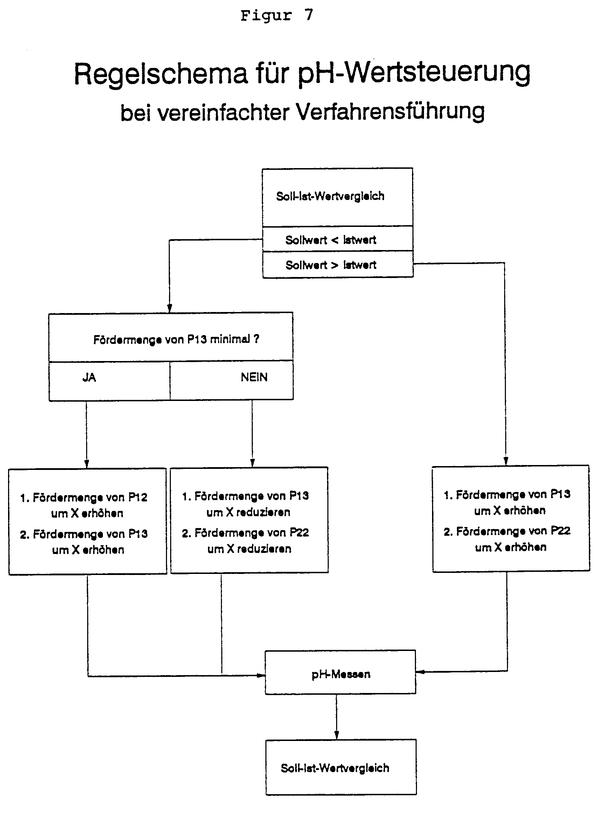

Ist allein eine Steuerung des pH-Wertes in der Feststoffhydrolyse maßgebend und/oder soll aus betriebstechnischen Gründen der maschinen- und steuerungstechnische Aufwand reduziert werden, kann die erfindungsgemäße Verfahrensführung und -Steuerung vereinfacht werden (siehe Fig. 6 und Fig. 7). Diese Verfahrensführung ermöglicht eine exakte Steuerung des pH-Wertes in der Feststoffhydrolyse und in Abhängigkeit der Durchmischung der Feststoffhydrolyse auch die Steuerung ihres Feststoffgehaltes.

In dieser Ausführungsform der Erfindung wird demnach das versäuerte Gemisch 12 vollständig der Feststoffhydrolyse zugeführt. Ein entsprechendes Volumen an Gemisch 13 wird aus dem Reaktor 2 zur Methanisierung geleitet. Überschüssiges Gemisch aus der Methanisierung 15 wird einer Fest/Flüssig-Trennung (FFT 6) zugeführt und Reststoffe 16 sowie Überschußwasser 23 getrennt aus dem Prozeß ausgeschleust.

Diese weitere, vereinfachte Ausführungsform der Erfindung verläuft somit ebenfalls in drei Stufen. Das feststoffhaltige Substrat wird in einem Reaktor 1 versäuert, dieses versäuerte Gemisch einem Reaktor 2 zur Hydrolysierung zugeführt und das hydrolysierte Gemisch anschließend einem Reaktor 3 zugeleitet, in welchem eine Methanisierung stattfindet. Erst das aus dem Reaktor 3 abgeleitete methanisierte Gemisch wird bevorzugt einer Fest/Flüssig-Trennung zugeführt. Eine Fest/Flüssig-Trennung zwischen dem Reaktor 1 und 2 und dem Reaktor 2 und 3 erfolgt nicht.

In dieser Ausführungsform der Erfindung entfallen die Trennvorrichtungen FFT 4 und FFT 5, d.h. ein Fest/Flüssig-Gemisch wird aus dem Reaktor 1 in den Reaktor 2 und von dort in den Reaktor 3 überführt. Erst das aus dem Reaktor 3 abgeleitete methanisierte Gemisch wird bevorzugt in eine Feststofffraktion und eine Flüssigstofffraktion getrennt.

Auch diese vereinfachte Ausführungsform der Erfindung ermöglicht eine pH-Wert-Steuerung, d.h. die pH-Werte der Reaktoren 2 und 3 können durch die Regelelemente P12 und P13 oder P13 und P22 gesteuert werden (vgl. Fig. 7).

Die pH-Wert-Steuerung erfolgt im einzelnen bevorzugt wie folgt:
Sinkt der pH-Wert im Reaktor 2 aufgrund der Menge an zugeführten oder im Reaktor gebildeten Säuren unter den optimalen Wert, wird zusätzlich Gemisch aus der Feststoffhydrolyse 13 der Methanisierung zugeführt und ein entsprechendes Volumen an methanisiertem Gemisch 22 in die Feststoffhydrolyse zurückgeführt. Dadurch werden methanogene, d.h. säureverwertende Mikroorganismen und Pufferkapazität der Feststoffhydrolyse zugeführt. Folglich steigt der pH-Wert im Reaktor 2.

Steigt der pH-Wert im Reaktor 2 zu weit, wird die Fördermenge an Gemisch aus der Methanisierung in die Feststoffhydrolyse 22 reduziert. Um den gleichen Betrag wird auch die Fördermenge an Gemisch von der Feststoffhydrolyse in die Methanisierung 13 verringert. Ist die Fördermenge von P22 Null und der pH-Wert im Reaktor 2 noch zu hoch, wird die Fördermenge an versäuertem Gemisch in die Feststoffhydrolyse P 12 erhöht. Ein Regelschema für die vereinfachte Verfahrensführung ist Fig. 7 zu entnehmen.

Der Feststoffgehalt in der Feststoffhydrolyse wird durch den Durchmischungsgrad des Reaktors (vollständig oder unvollständig) und Sedimentation bzw. Flotation der Feststoffe gesteuert. Der geringste Feststoffgehalt im Reaktor 2 stellt sich durch seine vollständige Durchmischung ein. Läßt man die Ausbildung eines Schlammbettes oder einer Schwimmdecke zu, kann die mittlere Feststoffkonzentration im Reaktor erhöht werden. Je mächtiger das Schlammbett oder die Schwimmdecke, desto höher die mittlere Feststoffkonzentration im Reaktor. Die Mächtigkeit des Schlammbettes oder der Schwimmdecke werden durch die Intensität der Durchmischung des Reaktors gesteuert.

Die Vorrichtung der vereinfachten Verfahrensführung ist aus Fig. 6 zu entnehmen.

Im wesentlichen weist diese Vorrichtung einen ersten Reaktor 1 für die Versäuerung der organischen Substanzen, einen zweiten Reaktor 2 für die Feststoffhydrolyse der angesäuerten organischen Substanzen und einen Reaktor 3 für die Methanisierung der organischen Substanzen auf.

Am Reaktor 1 ist ein Einlaß 10 angebracht, über welchen dieser mit den zu bearbeitenden organischen Substanzen beschickt wird.

Mit einem kommunizierenden Element 12, welches aus einer Leitung, einem Kanal oder ähnlichem bestehen kann, ist der Reaktor 1 mit dem Reaktor 2 verbunden.

Der Reaktor 2 ist durch das kommunizierende Element 13 mit dem Reaktor 3 verbunden. Der Reaktor 3 ist wiederum mit dem Reaktor 2 durch das kommunizierende Element 22 verbunden. Über Steuerungselemente (P12, P13, P22) ist die Zuführung bzw. Rückführung der gelösten und nicht gelösten Stoffe und damit auch der pH-Wert im Reaktor 2 und im Reaktor 3 steuerbar.

Über die Fest/Flüssig-Trennvorrichtung 6, die über das kommunizierende Element 15 mit dem Reaktor 3 verbunden ist, wird der Methanisierungsablauf in die festen Feststoffe und das flüssige Überschußwasser aufgetrennt.

Die Reaktoren 2 und 3 weisen weiterhin abführende Elemente 30, 31 für die Entnahme von Gasen auf.

Das erfindungsgemäße Verfahren und die erfindungsgemäße Vorrichtung ermöglichen beispielsweise sowohl einen thermophilen als auch einen mesophilen Betrieb, wobei beispielsweise volldurchmischte Reaktoren, aber auch andere, dem Fachmann bekannte Reaktoren verwendbar sind.

Im Vergleich zum PAQUES-Verfahren und der in den Figuren 1 - 3 beschriebenen Verfahrensführung weist die in den Figuren 6 und 7 beschriebene vereinfachte Verfahrensführung folgende Vorteile auf:
Im Prozeß ist keine mechanische Fest/Flüssig-Trennung notwendig. Diese zeigt sich bei vielen Substraten als äußerst problematisch; entweder ist die Durchsatzleistung der Aggregate zu gering, oder der Feststoffrückhalt ungenügend oder die Betriebskosten durch Einsatz von Hilfsmitteln zu hoch.

Im beschriebenen Verfahren können alle drei Reaktoren als volldurchmischte Reaktoren ausgelegt werden. Diese sind unempfindlicher gegenüber Kalkablagerungen. Bei thermophilem Betrieb entfällt die hygienisierende Stufe. Der steuerungstechnische Aufwand ist deutlich reduziert.

## Patentansprüche

1. Verfahren zur biologischen Aufbereitung organischer Substanzen, insbesondere zur anaeroben biologischen Hydrolyse für die anschließende Biomethanisierung, mit pH-Wert-Steuerung, bei welchem die einem ersten Reaktor (1) zugeführten gelösten und/oder nichtgelösten organischen Substanzen in diesem Reaktor (1) zumindest einer Versäuerung unterzogen werden, der Hauptteil der dem ersten Reaktor (1) direkt entnommenen nichtgelösten zumindest angesäuerten organischen Substanzen einem zweiten Reaktor (2) für die Durchführung zumindest einer Feststoffhydrolyse zugeführt wird, wobei keine Rückführung der nichtgelösten Substanzen in den ersten Reaktor (1) erfolgt, und gelöste und nichtgelöste organische Substanzen dem Reaktor (2) aus dem Reaktor (1) direkt zugeführt werden, und der Hauptteil der gelösten zumindest angesäuerten organischen Substanzen aus dem ersten Reaktor (1) und aus dem zweiten Reaktor (2) einem dritten Reaktor (3) für die Durchführung zumindest einer Methanisierung zugeführt wird und zur Steuerung des pH-Wertes im Reaktor (2) die Stoffstrommenge aus dem Reaktor (1) in den Reaktor (2) und in den Reaktor (3) und/oder aus dem Reaktor (2) in den Reaktor (3) reguliert wird.

2. Verfahren nach Anspruch 1,
dadurch gekennzeichnet,
daß ein Hauptteil der dem ersten Reaktor (1) entnommenen organischen Substanzen einer Trennung in eine Feststofffraktion, welche im wesentlichen die nichtgelösten organischen Substanzen enthält, und in eine Flüssigfraktion, welche im wesentlichen die gelösten organischen Substanzen enthält, unterzogen wird.

3. Verfahren nach Anspruch 1 oder 2,
dadurch gekennzeichnet,
daß dem Reaktor (1) gelöste und nichtgelöste organische Substanzen entnommen und zur Steuerung, insbesondere zur Senkung, des pH-Wertes dem zweiten Reaktor (2) zugeführt werden.

4. Verfahren nach Anspruch 1, 2 oder 3,
dadurch gekennzeichnet,
daß dem zweiten Reaktor (2) gelöste und nichtgelöste organische Substanzen entnommen werden und von diesen zumindest ein Teil der gelösten organischen Substanzen dem dritten Reaktor (3) zur Steuerung, insbesondere zur Erhöhung, des pH-Wertes des zweiten Reaktors (2) zugeführt werden, wobei der restliche Teil der entnommenen organischen Substanzen dem Reaktor (2) wieder zurückgeführt wird.

5. Verfahren nach Anspruch 1, 2, 3 oder 4,
dadurch gekennzeichnet,
daß der Feststoffgehalt im zweiten Reaktor (2) dadurch gesteuert wird, daß dem dritten Reaktor (3) ein feststoffarmer Stoffstrom entnommen und dem zweiten Reaktor (2) zugeführt wird.

6. Verfahren nach einem oder mehreren der vorhergehenden Ansprüche,
dadurch gekennzeichnet,
daß die pH-Wert-Steuerung über die Stellglieder P11, P12, und/oder P13 erfolgt.

7. Verfahren nach einem oder mehreren der vorstehenden Ansprüche,
dadurch gekennzeichnet,
daß überschüssiges Wasser dem dritten Reaktor (3) entnommen wird und dadurch das Verhältnis des dem ersten Reaktor (1) mit den organischen Substanzen zugeführten Wassers zu dem dem zweiten und dem dritten Reaktor (2, 3) entnommenen Wasser kompensiert wird.

8. Verfahren nach einem oder mehreren der vorhergehenden Ansprüche,
dadurch gekennzeichnet,
daß im zweiten Reaktor (2) zusätzlich zur Feststoffhydrolyse eine Methanisierung vorgenommen wird und dem zweiten Reaktor (2) und dem dritten Reaktor (3) Biogas, insbesondere Methangas, entnommen wird.

9. Verfahren nach einem oder mehreren der vorstehenden Ansprüche,
dadurch gekennzeichnet,
daß der erste Reaktor (1) kontinuierlich oder diskontinuierlich mit organischen Substanzen beschickt wird und von dem zweiten Reaktor (2) kontinuierlich gelöste organische Substanzen dem dritten Reaktor (3) zugeführt werden und kontinuierlich dem zweiten Reaktor (2) Reststoffe entnommen werden, wobei die Reststoffe vorzugsweise durch Trennung der nichtgelösten organischen Substanzen von den gelösten organischen Substanzen nach der Entnahme aus dem zweiten Reaktor (2) vor der Beschickung des dritten Reaktors (3) gewonnen werden.

10. Verfahren nach einem oder mehreren der vorhergehenden Ansprüche,
dadurch gekennzeichnet,
daß der erste Reaktor (1) kontinuierlich oder diskontinuierlich mit organischen Substanzen beschickt wird und der zweite Reaktor (2) diskontinuierlich beschickt und ihm diskontinuierlich Reststoffe entnommen werden.

11. Verfahren nach einem oder mehreren der vorhergehenden Ansprüche,
dadurch gekennzeichnet,
daß zur pH-Wert-Absenkung folgende Schritte ausgeführt werden:
a) die Rückführung (13) des Inhalts aus Reaktor (2) zur FFT (4) durch Verringern der Fördermenge von P13 verringert wird.
b) durch Anpassung der Fördermenge von P22 an die produzierte Flüssigphase (F20) entsprechend dem vorgegebenen Verhältnis wird der Feststoffgehalt im Reaktor (2) konstant gehalten.

12. Verfahren nach einem oder mehreren der vorhergehenden Ansprüche,
dadurch gekennzeichnet,
daß, wenn die Fördermenge von P13 minimal oder Null ist, zur weiteren pH-Wert-Absenkung folgende Schritte ausgeführt werden:
a) versäuertes Gemisch wird über (12) direkt dem Reaktor (2) zugeführt,
b) die Zufuhr aus (11) wird gesenkt,
c) die Fördermenge von P12 wird erhöht,
d) die Fördermenge von P11 wird entsprechend gesenkt und
e) durch Anpassung der Fördermenge von P22 an die produzierte Flüssigphase (F20) entsprechend dem vorgegebenen Verhältnis wird der Feststoffgehalt im Reaktor (2) konstant gehalten.

13. Verfahren nach einem oder mehreren der vorhergehenden Ansprüche,
dadurch gekennzeichnet,
daß zur Anhebung des pH-Werts folgende Schritte durchgeführt werden:
a) die Zufuhr des versäuerten Gemisches über (11) wird erhöht und die von (12) entsprechend gesenkt;
b) die Fördermenge von P11 wird erhöht und die von P12 entsprechend gesenkt;
c) durch Anpassung der Fördermenge von P22 an die produzierte Flüssigphase (F20) entsprechend dem vorgegebenen Verhältnis wird der Feststoffgehalt im Reaktor (2) konstant gehalten.

14. Verfahren nach einem oder mehreren der vorhergehenden Ansprüche,
dadurch gekennzeichnet,
daß zur weiteren Anhebung des pH-Wertes folgende Schritte ausgeführt werden:
a) die Fördermenge von P13 über die Leitung 13 wird erhöht;
b) durch Anpassung der Fördermenge von P22 an die produzierte Flüssigphase (F20) entsprechend dem vorgegebenen Verhältnis wird der Feststoffgehalt im Reaktor (2) konstant gehalten.

15. Verfahren zur biologischen Aufbereitung organischer Substanzen, insbesondere zur anaeroben biologischen Hydrolyse für die anschließende Biomethanisierung, mit pH-Wert-Steuerung, bei welchem die einem ersten Reaktor (1) zugeführten gelösten und/oder nichtgelösten organischen Substanzen in diesem Reaktor (1) zumindest einer Versäuerung unterzogen werden, das versäuerte, die festen und die flüssigen organischen Substanzen enthaltende, direkt entnommene Gemisch im wesentlichen vollständig und direkt einem zweiten Reaktor (2) zur Durchführung zumindest einer Feststoffhydrolyse zugeführt wird, wobei keine Rückführung der nichtgelösten Substanzen in den Reaktor (1) erfolgt und das dem Reaktor (2) entnommene, feste und flüsige Bestandteile enthaltende, zumindest partiell hydrolysierte Gemisch im wesentlichen vollständig einem dritten Reaktor (3) zur Durchführung zumindest einer Methanisierung zugeführt wird und zur Steuerung des pH-Wertes im Reaktor (2) die Stoffstrommenge aus dem Reaktor (1) in den Reaktor (2) und aus dem Reaktor (2) in den Reaktor (3) oder aus dem Reaktor (2) in den Reaktor (3) und aus dem Reaktor (3) in den Reaktor (2) reguliert wird.

16. Verfahren nach Anspruch 15,
dadurch gekennzeichnet,
daß überschüssiges Gemisch aus der Methanisierung (15) einer Fest/Flüssig-Trennvorrichtung (6) zugeführt wird, wodurch Reststoffe (16) und Überschußwasser (23) getrennt aus dem Verfahren ausgeschleust werden.

17. Verfahren nach Anspruch 15 oder 16,
dadurch gekennzeichnet,
daß die pH-Wert-Steuerung über die Stellglieder P12 und P13 oder P13 und P22 erfolgt.

18. Verfahren nach einem oder mehreren der vorhergehenden Ansprüche 15 - 17,
dadurch gekennzeichnet,
daß zur pH-Wert-Anhebung im Reaktor (2) die nachfolgenden Schritte ausgeführt werden:
a) Abführung von zusätzlichem Gemisch aus der Feststoffhydrolyse (13) in den Reaktor (3)
b) Rückführung einer entsprechenden Menge an methanisiertem Gemisch (22) in den Reaktor (2).

19. Verfahren nach einem oder mehreren der vorhergehenden Ansprüche 15 - 18,
dadurch gekennzeichnet,
daß zur pH-Wert Absenkung im Reaktor (2) die nachfolgenden Schritte ausgeführt werden:
a) Verringerung der Menge des aus dem Reaktor (3) in den Reaktor (2) abgeführten Gemisches (22)
b) Verringerung des aus dem Reaktor (2) in den Reaktor (3) abgeführten Gemisches (13) in entsprechender Menge.

20. Verfahren nach einem oder mehreren der vorhergehenden Ansprüche 15 - 19,
dadurch gekennzeichnet,
daß zur weiteren pH-Wert Absenkung im Reaktor (2) die Fördermenge an versäuertem Gemisch (12) in den Reaktor (2) erhöht wird.

21. Verfahren nach einem oder mehreren der vorhergehenden Ansprüche 15 - 20,
dadurch gekennzeichnet,
daß der Feststoffgehalt über den Durchmischungsgrad des Reaktors (2) gesteuert wird, wobei
a) bei vollständiger Durchmischung der Feststoffgehalt vermindert oder
b) bei abnehmender Durchmischung der Feststoffgehalt erhöht werden kann.

22. Verfahren nach einem oder mehreren der vorhergehenden Ansprüche 15 - 21,
dadurch gekennzeichnet,
daß im Zweiten Reaktor (2) zusätzlich zur Feststoffhydrolyse eine Methanisierung vorgenommen wird und dem zweiten Reaktor (2) und dem dritten Reaktor (3) Biogas, insbesondere Methangas, entnommen wird.

23. Vorrichtung zur Durchführung eines Verfahrens nach einem oder mehreren der Ansprüche 15 - 22,
dadurch gekennzeichnet,
daß ein erster mit einer Zuführung (10) versehener Reaktor (1) über ein kommunizierendes Element (12) mit einem zweiten Reaktor (2), und dieser zweite Reaktor über ein kommunizierendes Element (13) und wahlweise zusätzlich einem kommunizierenden Element (22) mit einem dritten Reaktor (3) verbunden ist wobei kein Element zur Rückführung der Feststoffe in den Reaktor (1) vorgesehen ist, und zur PH-Wert-Steuerung im Reaktor (2) die Stellglieder P12 und P13 oder P13 und P22 vorgesehen sind und am Reaktor (3) ein Auslaß (31) für die Entnahme von Gasen angebracht ist.

24. Vorrichtung nach Anspruch 23,
dadurch gekennzeichnet,
daß der Reaktor (3) über ein kommunizierendes Element (15) mit einer Fest/Flüssig-Trennvorrichtung (6) verbunden ist.

25. Vorrichtung nach Anspruch 23 oder 24,
dadurch gekennzeichnet,
daß am zweiten Reaktor (2) ein Auslaß (30) für die Entnahme von Gasen angebracht ist.

26. Vorrichtung nach einem oder mehreren der vorhergehenden Ansprüche 23 - 25,
dadurch gekennzeichnet,
daß der Reaktor (2) ein durchmischter oder ein plug-flow-Reaktor ist.

27. Vorrichtung nach einem oder mehreren der vorhergehenden Ansprüche 23 - 26,
dadurch gekennzeichnet,
daß der Reaktor (3) ein durchmischter Reaktor ist.

28. Vorrichtung nach einem oder mehreren der vorhergehenden Ansprüche 23 - 27,
dadurch gekennzeichnet,
daß die Reaktoren (2) und/oder (3) kaskadenförmig ausgebildet sind.

29. Vorrichtung zur Durchführung eines Verfahrens nach einem oder mehreren der vorhergehenden Ansprüche 1 - 14,
dadurch gekennzeichet,
daß ein erster mit einer Zuführung (10) versehener Reaktor (1) über kommunizierende Elemente (11, 14), die über eine Trennvorrichtung (4) für die Trennung gelöster und nichtgelöster Stoffe in eine Feststofffraktion und eine Flüssigfraktion miteinander verbunden sind, mit einem zweiten Reaktor (2) verbunden ist, und die Trennvorrichtung (4) über das kommunizierende Element (20) mit einem dritten Reaktor (3) verbunden ist wobei kein Element zur Rückführung der Feststoffe in den Reaktor (1) vorgesehen ist und zur pH-Wert-Steuerung im Reaktor (2) die Stellglieder P11, P12 und/oder P13 vorgesehen sind und am Reaktor (3) ein Auslaß (31) für die Entnahme von Gasen angebracht ist.

30. Vorrichtung nach Anspruch 29,
dadurch gekennzeichnet,
daß der erste Reaktor (1) über ein weiteres kommunizierendes Element (12) mit einem Stellglied P12 mit dem zweiten Reaktor (2) verbunden ist.

31. Vorrichtung nach einem oder mehreren der vorhergehenden Ansprüche 29 - 30,
dadurch gekennzeichnet,
daß der dritte Reaktor (3) über ein weiteres kommunizierendes Element (22) mit einem Stellglied P22 mit dem zweiten Reaktor (2) direkt und/oder über das Element (14) verbunden ist.

32. Vorrichtung nach einem oder mehreren der vorhergehenden Ansprüche 29 - 31,
dadurch gekennzeichnet,
daß an dem dritten Reaktor (3) ein Auslaß (23) für die Entnahme von Wasser angeordnet ist.

33. Vorrichtung nach einem oder mehreren der vorhergehenden Ansprüche 29 - 32,
dadurch gekennzeichnet,
daß der zweite Reaktor (2) über ein kommunizierendes Element (15) mit einer Trennvorrichtung (5) für die Trennung gelöster und nichtgelöster Stoffe in eine Feststofffraktion und in eine Flüssigfraktion verbunden ist, wobei der Ausgang für die Flüssigfraktion der Trennvorrichtung (5) über die kommunizierenden Elemente (21) und (20) mit dem dritten Reaktor (3) verbunden ist, und die Trennvorrichtung (5) einen Auslaß (16) für Feststoffe aufweist.

34. Vorrichtung nach einem oder mehreren der vorhergehenden Ansprüche 29 - 33,
dadurch gekennzeichnet,
daß am zweiten Reaktor (2) ein Auslaß (30) für die Entnahme von Gasen angebracht ist.

35. Vorrichtung nach einem oder mehreren der vorhergehenden Ansprüche 29 - 34,
dadurch gekennzeichnet,
daß der Reaktor (2) ein durchmischter oder ein plug-flow-Reaktor ist.

36. Vorrichtung nach einem oder mehreren der vorhergehenden Ansprüche 29 - 35,
dadurch gekennzeichnet,
daß der Reaktor (3) ein Festbettreaktor ist.

37. Vorrichtung nach einem oder mehreren der vorhergehenden Ansprüche 29 - 36,
dadurch gekennzeichnet,
daß die Reaktoren (2) und/oder (3) kaskadenförmig ausgebildet sind.

## Claims

1. A method for the biological processing of organic substances and more particularly for anaerobic biological hydrolysis for subsequent biomethanization, with pH value control, in the case of which the dissolved and/or undissolved organic substances supplied to a first reactor (1) are at least subjected to an acidification in said reactor (1), the major part of the undissolved, at least acidified organic substances directly taken from the first reactor (1) are supplied to a second reactor (2) for the performance of at least one solids hydrolysis step, wherein no return of undissolved substances into said first reactor (1) is performed, and dissolved and undissolved organic substances are directly supplied to reactor (2) from said reactor (1), and the main part of the dissolved, at least acidified organic substances from said first reactor (1) and from said second reactor (2) are supplied to a third reactor (3) for the performance of at least one methanization step, and wherein the amount of flow of material from said reactor (1) into said reactor (2) and into said reactor (3) and/or from said reactor (2) into said reactor (3) is regulated for controlling the pH value in said reactor (2).

2. Method according to claim 1,
characterized in that
a major part of the organic substances taken from said first reactor (1) is subjected to separation into a solids fraction, which essentially comprises the undissolved organic substances, and into a liquid fraction, which essentially comprises the dissolved organic substances.

3. Method according to claim 1 or 2,
characterized in that
dissolved and undissolved organic substances are taken from said reactor (1) and are supplied to said second reactor (2) for pH value control and more particularly for reducing the pH value.

4. Method according to claim 1, 2 or 3,
characterized in that
dissolved and undissolved organic substances are taken from said second reactor (2) and from them at least a part of the dissolved organic substances is supplied to said third reactor (3) for pH value control, and more particularly for increasing the pH value in said second reactor (2), the remaining part of the drawn off organic substances being returned to said reactor (2).

5. Method according to claim 1, 2, 3 or 4,
characterized in that
the solids content in said second reactor (2) is controlled by removing a low solids feed from the third reactor (3) and supplying it to said second reactor (2).

6. Method according to any of the preceding claims,
characterized in that
pH value control is performed using the servo members P11, P12 and/or P13.

7. Method according to any of the preceding claims,
characterized in that
excess water is taken from said third reactor (3) and in this manner the ratio between the water supplied to said first reactor (1) with the organic substances and the water taken from said second and third reactors (2, 3) is compensated for.

8. Method according to any of the preceding claims,
characterized in that
in said second reactor in addition to solids hydrolysis methanization is performed and biogas, more particularly in the form of methane, is removed from said second reactor (2) and said third reactor (3).

9. Method according to any of the preceding claims,
characterized in that
said first reactor (1) is continuously or discontinuously charged with organic substances and dissolved organic substances are continuously supplied from said second reactor (2) to said third reactor (3) and continuously residual materials are removed from said second reactor (2), the residual substances being preferably obtained by separation of the undissolved organic substances from the dissolved organic substances after removal from said second reactor (2) prior to charging said third reactor (3).

10. Method according to any of the preceding claims,
characterized in that
said first reactor (1) is continuously or discontinuously charged with organic substances and said second reactor (2) is discontinuously charged and residual substances are discontinuously removed from it.

11. Method according to any of the preceding claims,
characterized in that
for reduction of the pH value the following steps are performed:
a) return (13) of the content of said reactor (2) to FFT (4) by reduction of the pumping rate of P13;
b) by adapting the pumping rate of P22 to the produced fluid phase (F20) in accordance with the predetermined ratio, the solids content in said reactor (2) is maintained constant.

12. Method according to any of the preceding claims,
characterized in that
when the pumping rate (13) is at a minimum or zero, the following steps are performed for further reduction of the pH value:
a) acidified mixture is supplied via (12) directly to said reactor (2),
b) the supply from (11) is reduced,
c) the pumping rate of P12 is increased,
d) the pumping rate of P11 is correspondingly reduced and
e) by adaptation of the pumping rate of P22 to the liquid phase (F20) produced in accordance with the predetermined ratio, the solids content in said reactor (2) is maintained constant.

13. Method according to any of the preceding claims,
characterized in that
for increasing the pH value the following steps are performed:
a) the supply of the acidified mixture via (11) is increased and the supply from (12) is correspondingly lowered;
b) the pumping rate of P11 is increased and the rate of P12 is correspondingly reduced;
c) by adaptation of the pumping rate of P22 to the produced fluid phase (F20) in accordance with the predetermined ratio, the solids content in said reactor (2) is maintained constant.

14. Method according to any of the preceding claims,
characterized in that
for further increasing the pH value, the following steps are performed:
a) the pumping rate of P13 is increased via the duct (13);
b) by adaptation of the pumping rate of P22 to the produced fluid phase (F20) in accordance with the predetermined ratio, the solids content in said reactor (2) is maintained constant.

15. A method for the biological processing of organic substances, in particular for anaerobic biological hydrolysis for subsequent biomethanization, with pH value control, in the case of which the dissolved and/or undissolved organic substances supplied to the first reactor (1) are at least subjected to an acidification in said reactor (1), the acidified directly taken mixture containing the solid and liquid organic substances is substantially completely and directly supplied to a second reactor (2) for the performance of at least one solids hydrolysis step, wherein no return of undissolved substances into said reactor (1) is performed, and the at least partly hydrolysized mixture taken from said reactor (2) and containing solid and liquid components is substantially completely supplied to a third reactor (3) for the performance of at least one methanization step and for pH value control in said reactor (2) the amount of flow of material from said reactor (1) into said reactor (2) and from said reactor (2) into said reactor (3) or from said reactor (2) into said reactor (3) and from said reactor (3) into said reactor (2) is regulated.

16. Method according to claim 15,
characterized in that
excess mixture from the methanization step (15) is supplied to a solids/liquids separating device (6) with the result that the residual materials (16) and excess water (23) are drawn off separately from the process.

17. Method according to claim 15 or 16,
characterized in that
the pH value control is performed using the servo members P12 and P13 or P13 and P22.

18. Method according to one or more of the preceding claims 15 - 17,
characterized in that
in said reactor (2) the following steps are performed for increasing the pH value:
a) removal of additional mixture from the solids hydrolysis (13) into said reactor (3);
b) return of a corresponding quantity of methanized mixture (22) into said reactor (2).

19. Method according to one or more of the preceding claims 15 - 18,
characterized in that
for reduction of the pH value in said reactor (2) the following steps are performed:
a) reduction of the quantity of the mixture (22) removed from said reactor (3) into said reactor (2);
b) a corresponding reduction of the quantity of mixture (13) taken from said reactor (2) into said reactor (3).

20. Method according to one or more of the preceding claims 15 - 19,
characterized in that
for further reduction of the pH value in said reactor (2), the pumping rate of the acidified mixture (12) into said reactor (2) is increased.

21. Method according to one or more of the preceding claims 15 - 20,
characterized in that
the solids content is controlled via the degree of mixing in said reactor (2), wherein
a) in the case of a complete mixing the solids content may be reduced or
b) in the case of a reduction in mixing the solids content may be increased.

22. Method according to one or more of the preceding claims 15 - 21,
characterized in that
in said second reactor (2) in addition to solids hydrolysis methanization is performed and biogas, more particular methane, is removed from said second reactor (2) and said second reactor (3).

23. An apparatus for performing a method as claimed in one or more of the preceding claims 15 - 22,
characterized in that
a first reactor (1) provided with a supply means (10) is connected with a second reactor (2) via a communicating element (12), and said second reactor is connected via a communicating element (13) and an optionally additionally a communicating element (22) with a third reactor (3), wherein no element for recycling these solids into said reactor (1) is provided, and for pH value control in reactor (2) the servo members P12 and P13 or P13 and P22 are provided and an outlet (31) is provided on said reactor (3) for removal of gases.

24. Apparatus according to claim 23,
characterized in that
said reactor (3) is connected via a communicating element (15) with a solids/liquid separating device (6).

25. Apparatus according to claim 23 or 24,
characterized in that
said second reactor (2) is provided with an outlet (30) for the removal of gases.

26. Apparatus according to one or more of the preceding claims 23 - 25,
characterized in that
said reactor (2) is adapted for full mixing of the contents thereof or is a plug-flow reactor.

27. Apparatus according to one or more of the preceding claims 23 - 26,
characterized in that
said reactor (3) is adapted for full mixing of the contents.

28. Apparatus according to one or more of the preceding claims 23 - 27,
characterized in that
said reactors (2) and/or (3) are arranged in cascade.

29. Apparatus for performing a method according to one or more of the preceding claims 1 - 14,
characterized in that
the first reactor (1) provided with a supply means (10) is connected via communicating elements (11, 14), which are connected with each other via a separating device (4) for the separation of dissolved and non-dissolved materials into a solids fraction and into a liquid fraction, with a second reactor (2), and the separating device (4) is connected via the communicating element (20) with a third reactor (3) without any element for return of the solids into said reactor (1) is provided and for pH value control in said reactor (2) servo members P11, P12 and/or P13 are provided and an outlet (31) is provided on said reactor (3) for removal of gases.

30. Apparatus according to claim 29,
characterized in that
said first reactor is connected via a further communicating element (12) with a servo member P12 with said second reactor (2).

31. Apparatus according to one or more of the preceding claims 29 - 30,
characterized in that
said third reactor (3) is connected via a further communicating element (22) with a servo member P22 directly/or via the element (14) with said second reactor (2).

32. Apparatus according to one or more of the preceding claims 29 - 31,
characterized in that
an outlet (23) is provided on said third reactor (3) for the removal of water.

33. Apparatus according to one or more of the preceding claims 29 - 32,
characterized in that
said second reactor (2) is connected via a communicating element (15) with a separating device (5) for the separation of dissolved and non-dissolved materials into a solids fraction and a liquid fraction, the outlet for the liquid fraction of the separating device (5) is connected via communicating elements (21) and (20) with said third reactor (3), and the separating device (5) is provided with an outlet (16) for solids material.

34. Apparatus according to one or more of the preceding claims 29 - 33,
characterized in that
an outlet (30) for the removal of gases is provided on said second reactor (2).

35. Apparatus according to one or more of the preceding claims 29 - 34,
characterized in that
said second reactor (2) is adapted for full mixing of the contents thereof or is a plug-flow reactor.

36. Apparatus according to one or more of the preceding claims 29 - 35,
characterized in that
said reactor (3) is a fixed bed reactor.

37. Apparatus according to one or more of the preceding claims 29 - 36,
characterized in that
said reactors (2) or (3) are connected in cascade.

## Revendications

1. Procédé pour le traitement biologique de substances organiques, en particulier pour l'hydrolyse biologique anaérobie à la biométhanisation suivante, avec contrôle du pH, pour lequel les substances organiques dissoutes et/ou non dissoutes amenées à un premier réacteur (1) sont soumises dans ce réacteur (1) au moins à une acidification; la partie principale des substances organiques non dissoutes, au moins acidifiées et prelevées directement dans le premier réacteur (1) est amenée à un deuxième réacteur (2) pour l'exécution d'au moins une hydrolyse de solides, aucun recyclage des substances non dissoutes dans le premier réacteur (1) ne se produisant et des substances organiques dissoutes et non dissoutes étant amenées directement du réacteur (1) au réacteur (2), et la majeure partie des substances organiques, dissoutes et au moins acidifiées, provenant du premier réacteur (1) et du deuxième réacteur (2) est amenée à un troisième réacteur (3) pour la réalisation d'au moins une méthanisation et, pour contrôler le pH dans le réacteur (2), la quantité du flux de produit sortant du réacteur (1) pour entrer dans le réacteur (2) et dans le réacteur (3) et/ou sortant du réacteur (2) pour entrer dans le réacteur (3) est régularisée.

2. Procédé selon la revendication 1, caractérisé en ce qu'une partie principale des substances organiques prélevées dans le premier réacteur (1) est soumise à une séparation en une fraction solide, qui contient essentiellement les substances organiques non dissoutes, et une fraction liquide, qui contient essentiellement les substances organiques dissoutes.

3. Procédé selon la revendication 1 ou 2, caractérisé en ce que des substances organiques dissoutes et non dissoutes sont prélevées dans le réacteur (1) et sont amenées au deuxième réacteur (2) pour le contrôle, en particulier pour la diminution, de la valeur pH.

4. Procédé selon l'une quelconque des revendications 1, 2 ou 3, caractérisé en ce que des substances organiques dissoutes et non dissoutes sont prélevées dans le deuxième réacteur (2) et que, a partir de celles-ci, au moins une partie des substances organiques dissoutes est amenée au troisième réacteur (3) pour le contrôle, en particulier pour l'augmentation, de la valeur pH du deuxième réacteur (2), la partie résiduelle des substances organiques prélevées étant ramenée au réacteur (2).

5. Procédé selon l'une quelconque des revendications 1, 2, 3 ou 4, caractérisé en ce que la teneur de matières solides dans le deuxième réacteur (2) est contrôlée par le fait qu'un flux de produit pauvre en matières solides est prélevée dans le troisième réacteur (3) et amenée au deuxième réacteur (2).

6. Procédé selon l'une quelconque ou plusieurs des revendications précédentes, caractérisé en ce que le contrôle du pH s'effectue par les actionneurs P11, P12 et/ou P13.

7. Procédé selon l'une quelconque ou plusieurs des revendications précédentes, caractérisé en ce que de l'eau excédentaire est prélevée dans le troisième réacteur (3) et que de ce fait le rapport de proportion entre l'eau amenée au premier réacteur (1) avec les substances organiques et l'eau amenée aux deuxième et troisième réacteurs (2, 3) est compensé.

8. Procédé selon l'une ou plusieurs des revendications précédentes, caractérisé en ce qu'une méthanisation est effectuée dans le deuxième réacteur (2) en supplément de l'hydrolyse des solides et du biogaz, en particulier du gaz méthane, est prélevé dans le deuxième réacteur (2) et le troisième réacteur (3).

9. Procédé selon l'une quelconque ou plusieurs des revendications précédentes, caractérisé en ce que le premier réacteur (1) est alimenté de façon continue ou discontinue avec des substances organiques, des substances organiques dissoutes sont amenées en continu du deuxième réacteur (2) au troisième réacteur (3) et des substances résiduelles sont prélevées en continu dans le deuxième réacteur (2), les substances résiduelles étant obtenues de préférence par séparation des substances organiques non dissoutes des substances organiques dissoutes après le prélèvement dans le deuxième réacteur (2) et avant l'alimentation du troisième réacteur (3).

10. Procédé selon l'une quelconque ou plusieurs des revendications précédentes, caractérisé en ce que le premier réacteur (1) est alimenté de façon continue ou discontinue avec des substances organiques et le deuxième réacteur (2) est alimenté de façon discontinue et des substances résiduelles sont prélevées dans ce reacteur de façon discontinue.

11. Procédé selon l'une quelconque ou plusieurs des revendications précédentes, caractérisé en ce que l'on exécute les étapes suivantes pour abaisser la valeur pH :
a) le recyclage (13) du contenu du réacteur (2) vers le FFT (4) est réduit par l'abaissement du débit de P13 ;
b) par l'adaptation du débit de P22 à la phase liquide (F20) produite en fonction du rapport de proportion prédéfini
c) la teneur de matières solides est maintenue à un niveau constant dans le réacteur (2).

12. Procédé selon l'une quelconque ou plusieurs des revendications précédentes, caractérisé en ce que le débit de P13 est minimal ou nul et les étapes suivantes sont exécutées pour abaisser encore le pH :
a) le mélange acidifié est amené par (12) directement au réacteur (2),
b) la quantité arrivant de (11) est réduite,
c) le débit de P12 est augmenté,
d) le débit de P11 est abaissé en conséquence et
e) la teneur de matières solides est maintenue constante dans le réacteur (2) par adaptation du débit de P22 à la phase liquide (F20) produite en fonction du rapport de proportion prédéfini.

13. Procédé selon l'une quelconque ou plusieurs des revendications précédentes, caractérisé en ce qu'on exécute les étapes suivantes pour augmenter la valeur pH :
a) l'arrivée du mélange acidifié est augmentée par (11) et celle de (12) abaissée en conséquence ;
b) le débit de P11 est augmenté et celui de P12 abaissé en conséquence ;
c) la teneur de matières solides est maintenue constante dans le réacteur (2) par adaptation du débit de P22 à la phase liquide (F20) produite en fonction du rapport de proportion prédéfini.

14. Procédé selon l'une quelconque ou plusieurs des revendications précédentes, caractérisé en ce qu'on exécute les étapes suivantes pour augmenter encore la valeur pH
a) le débit de P13 est augmenté par la conduite 13 ;
b) la teneur de matières solides est maintenue constante dans le réacteur (2) par adaptation du débit de P22 à la phase liquide (F20) produite en fonction du rapport de proportion prédéfini.

15. Procédé pour le traitement biologique de substances organiques, en particulier pour l'hydrolyse biologique anaérobie à la biométhanisation suivante, avec contrôle du pH, pour lequel les substances organiques dissoutes et non dissoutes amenées à un premier réacteur (1) sont soumises dans ce réacteur (1) au moins à une acidification; le mélange acidifié, contenant les substances organiques solides et liquides et directement prélevé, est amené sensiblement complètement et directement à un deuxième réacteur (2) pour l'exécution d'au moins une hydrolyse de solides, aucun recyclage des substances non dissoutes dans le premier réacteur (1) ne se produisant, et le mélange prélevé dans le réacteur (2), contenant des composants solides et liquides, hydrolysé au moins partiellement, est amené sensiblement complètement à un troisième réacteur (3) pour la réalisation d'au moins une méthanisation et, pour contrôler le pH dans le réacteur (2), la quantité du flux de produit sortant du réacteur (1) pour entrer dans le réacteur (2) et sortant du réacteur (2) pour entrer dans le réacteur (3) ou sortant du réacteur (2) pour entrer dans le recyclage et sortant du réacteur (3) pour entrer dans le réacteur (2) est régularisée.

16. Procédé selon la revendication 15, caractérisé en ce que du mélange excédentaire provenant de la méthanisation (15) est amené à un dispositif de séparation solide/liquide (6), les produits résiduels (16) et l'eau excédentaire (23) étant éclusés séparément hors du procédé.

17. Procédé selon la revendication 15 ou 16, caractérisé en ce que le contrôle du pH s'effectue par les actionneurs P12 et P13 ou p13 et P22.

18. Procédé selon l'une quelconque ou plusieurs des revendications précédentes 15 à 17, caractérisé en ce qu'on exécute les étapes suivantes pour augmenter le pH dans le réacteur (2) :
a) évacuation de mélange supplémentaire de l'hydrolyse des solides (13) dans le réacteur (3)
b) recyclage d'une quantité appropriée de mélange (22) méthanisé dans le réacteur (2).

19. Procédé selon l'une quelconque ou plusieurs des revendications précédentes 15 à 18, caractérisé en ce qu'on exécute les étapes suivantes pour abaisser le pH dans le réacteur (2) :
a) réduction de la quantité du mélange (22) évacué du réacteur (3) dans le réacteur (2)
b) réduction du mélange (13) évacué du réacteur (2) dans le réacteur (3) dans une quantité appropriée.

20. Procédé selon l'une quelconque ou plusieurs des revendications précédentes 15 à 19, caractérisé en ce que, pour augmenter encore le pH dans le réacteur (2), on augmente le débit de mélange (12) acidifié transporté dans le réacteur (2).

21. Procédé selon l'une quelconque ou plusieurs des revendications précédentes 15 à 20, caractérisé en ce que la teneur de matières solides est contrôlée par le degré de mélangeage du réacteur (2),
a) la teneur de matières soldes pouvant être réduite avec un mélangeage complet ou
b) la teneur de matières solides pouvant être augmentée avec un degré de mélangeage en diminution.

22. Procédé selon l'une quelconque ou plusieurs des revendications précédentes 15 à 21, caractérisé en ce qu'une méthanisation est effectuée en plus de l'hydrolyse des solides dans le deuxième réacteur (2) et du biogaz, en particulier du gaz méthane, est prélevé dans le deuxième réacteur (2) et le troisième réacteur (3).

23. Dispositif pour l'application d'un procédé selon l'une quelconque ou plusieurs des revendications 15 à 22, caractérisé en ce qu'un premier réacteur (1) pourvu d'une arrivée (10) est relié à un deuxième réacteur (2) par un élément (12) communiquant, et ce deuxième réacteur est relié à un troisième réacteur (3) par un élément (13) communiquant et en option un élément (22) communiquant supplémentaire, aucun élément n'étant prévu pour le recyclage des solides dans le réacteur (1), et en ce que les actionneurs P12 et P13 ou P13 et P22 sont prévus pour contrôler le pH dans le réacteur (2) et une sortie (31) pour le prélèvement de gaz est disposée sur le réacteur (3).

24. Dispositif selon la revendication 23, caractérisé en ce que le réacteur (3) est relié par un élément (15) communiquant à un dispositif de séparation solide/liquide (6).

25. Dispositif selon la revendication 23 ou 24, caractérisé en ce qu'une sortie pour le prélèvement de gaz est disposée sur le deuxième réacteur (2).

26. Dispositif selort l'une quelconque ou plusieurs des revendications précédentes 23 à 25, caractérisée en ce que le réacteur (2) est un réacteur mixte ou plug-flow.

27. Dispositif selon l'une quelconque ou plusieurs des revendications précédentes 23 à 26, caractérisé en ce que le réacteur (3) est un réacteur mixte.

28. Dispositif selon l'une quelconque ou plusieurs des revendications précédentes 23 à 27, caractérisé en ce que les réacteurs (2) et/ou (3) sont constitués en forme de cascade.

29. Dispositif pour l'application du procédé selon l'une ou plusieurs des revendications précédentes 1 à 14, caractérisé en ce qu'un premier réacteur (1) équipé d'une arrivée (10) est relié à un deuxième réacteur (2) par des éléments (11, 14) communiquants, qui sont reliés entre eux par un dispositif de séparation (4) pour la séparation de substances dissoutes et non dissoutes en une fraction solide et une fraction liquide, et le dispositif de séparation (4) est relié à un troisième réacteur (3) par l'élément (20) communiquant, aucun élément n'étant prévu pour le recyclage des matières solides dans le réacteur (1), les actionneurs P11 et P12 et/ou P13 sont prévus pour le contrôle de la valeur pH dans le réacteur et une sortie (31) pour le prélèvement de gaz est disposée sur le réacteur (3).

30. Dispositif selon la revendication 29, caractérisé en ce que le premier réacteur (1) est relié par un autre élément (12) communiquant à un actionneur P12 au deuxième réacteur (2).

31. Dispositif selon l'une quelconque ou plusieurs des revendications précédentes 29 et 30, caractérisé en ce que le troisième réacteur (3) est relié par un autre élément (22) communiquant à un actionneur P22 au deuxième réacteur (2) directement et/ou par l'élément (14).

32. Dispositif selon l'une quelconque ou plusieurs des revendications précédentes 29 à 31, caractérisé en ce qu'une sortie (23) pour le prélèvement de l'eau est disposée sur le troisième réacteur (3).

33. Dispositif selon l'une quelconque ou plusieurs revendications précédentes 29 à 32, caractérisé en ce que le deuxième réacteur (2) est relié par un élément (15) communiquant à un dispositif de séparation (5) pour la séparation de substances dissoutes et non dissoutes en une fraction solide et en une fraction liquide, la sortie pour la fraction liquide du dispositif de séparation (5) étant relié par les éléments (21) et (20) communiquants au troisième réacteur (3), et le dispositif de séparation (5) présente une sortie (16) pour les solides.

34. Dispositif selon l'une quelconque ou plusieurs des revendications précédentes 29 à 33, caractérisé en ce qu'une sortie (30) pour le prélèvement de gaz est disposée sur le deuxième réacteur (2).

35. Réacteur selon l'une quelconque ou plusieurs des revendications précédentes 29 à 34, caractérisé en ce que le réacteur (2) est un réacteur mixte ou un réacteur plug-flow.

36. Dispositif selon l'une ou plusieurs des revendications 29 à 35, caractérisé en ce que le réacteur est un réacteur à lit fixe.

37. Dispositif selon l'une quelconque ou plusieurs des revendications précédentes 29 à 36, caractérisé en ce que les réacteurs (2) et/ou 3 sont conçus en forme de cascade.
